(19) Europäisches Patentamt
European Patent Office
Office européen des brevets

(11) **EP 4 517 323 A1**

(12) **EUROPEAN PATENT APPLICATION**
published in accordance with Art. 153(4) EPC

(43) Date of publication:
**05.03.2025 Bulletin 2025/10**

(21) Application number: **23807701.0**

(22) Date of filing: **18.05.2023**

(51) International Patent Classification (IPC):
***G01N 33/53*** (2006.01)  ***G01N 33/574*** (2006.01)
***G01N 21/53*** (2006.01)

(52) Cooperative Patent Classification (CPC):
**G01N 21/53; G01N 33/53; G01N 33/574**

(86) International application number:
**PCT/JP2023/018633**

(87) International publication number:
**WO 2023/224100 (23.11.2023 Gazette 2023/47)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **18.05.2022  JP 2022081826**

(71) Applicants:
• **University Public Corporation Osaka
Osaka City 545-0051 (JP)**
• **Aichi Prefecture
Aichi 460-8501 (JP)**

(72) Inventors:
• **IIDA, Takuya
Sakai-shi, Osaka 599-8531 (JP)**
• **TOKONAMI, Shiho
Sakai-shi, Osaka 599-8531 (JP)**
• **TAKAGI, Yumiko
Sakai-shi, Osaka 599-8531 (JP)**
• **NAKASE, Ikuhiko
Sakai-shi, Osaka 599-8531 (JP)**
• **TAGUCHI, Ayumu
Nagoya-shi, Aichi 464-8681 (JP)**

(74) Representative: **Prüfer & Partner mbB
Patentanwälte · Rechtsanwälte
Sohnckestraße 12
81479 München (DE)**

(54) **KIT FOR DETECTING GLYCOPROTEIN**

(57)     Provided is a kit for detecting a glycoprotein contained in a sample using an optical condensation system, the kit comprising microparticles modified by host molecules and a dilution solution for diluting the sample, in which each of the host molecules binds specifically to the glycoprotein, the dilution solution comprises a blocking agent and a buffering agent, the pH value of the dilution solution is higher than the isoelectric point of the glycoprotein, the concentration of the blocking agent is lower than a concentration at which the non-specific adsorption between the host molecules is inhibited in an environment where a light-induced force does not act on the host molecules, and the salt concentration in the dilution solution is a concentration at which the microparticles modified by the host molecules cannot be precipitated by salting out.

FIG.3

EP 4 517 323 A1

**Description**

TECHNICAL FIELD

The present invention relates to a kit for detecting a glycoprotein.

BACKGROUND ART

Various technologies are in practical use to detect analytes that may be contained in the sample. Examples of the analytes include allergens, proteins derived from cancer cells (for example, glycoproteins derived from cancer cells), nucleic acids, and vesicles. For example, the ELISA (Enzyme-Linked Immuno Sorbent Assay) method or the SPR (Surface Plasmon Resonance) method are known as protein detection techniques. The lowest concentration (detection limit) of the analyte by the ELISA method is considered to be about 0.3 ng/mL. The detection limit of the SPR method is considered to be about 1 $\mu$g/mL. In addition, both methods require several hours to detect the analyte.

CITATION LIST

PATENT LITERATURE

PTL 1: WO 2014/192937
PTL 2: WO 2021/040021

SUMMARY OF INVENTION

TECHNICAL PROBLEM

In recent years, technologies for detecting analytes by utilizing the action of light, such as light-induced forces, have been attracting attention. For example, WO 2014/192937 (Patent Literature 1) discloses a detection device for detecting an analyte that may be contained in a sample, the device comprising: a plurality of metallic nanoparticles modified by host molecules, each of which can attach specifically to the analyte; a first light source that emits polarized light for assembling the plurality of metallic nanoparticles; an objective lens that focuses the polarized light and introduces the focused polarized light into a liquid containing the sample and the plurality of metallic nanoparticles; a light receiver that receives light from the liquid; a detector that detects the analyte based on signals from the light receiver.
Also, WO 2021/040021 (Patent Literature 2) discloses a method for detecting an analyte, the method comprising: distributing a liquid sample containing a plurality of microparticles modified by host molecules, each of which binds specifically to the analyte, in a microchannel using a pump; irradiating the liquid sample with non-resonant light that is light outside the electronically resonant wavelength region of the plurality of microparticles; and detecting the analyte based on signals from a light receiver that receives light from the liquid sample.
There is always a demand for technologies that increase the detection sensitivity for an analyte or reduce the detection time for an analyte, in other words, technologies that enable rapid detection of a minute amount of an analyte. In particular, from the viewpoints of early diagnosis technology for cancer, medical checkups, and development of in vitro diagnostic pharmaceuticals (such as general diagnostic agents and medical diagnostic agents), for example, there is a need to shorten detection time and improve detection sensitivity in the technology for detecting glycoproteins.
The present invention has been made in view of the above circumstances, and an object of the present invention is to provide a kit for detecting a minute amount of a glycoprotein contained in a sample rapidly and with high sensitivity using an optical condensation system.

SOLUTION TO PROBLEM

As a result of diligent studies in order to solve the above problem, the present inventors have found that by optimizing a dilution solution for diluting a sample, a minute amount of a glycoprotein contained in the sample is detected rapidly and with high sensitivity using an optical condensation system, thereby completing the present invention. That is, the present invention is as follows.

[1] The kit according to the present invention is a kit for detecting a glycoprotein contained in a sample using an optical condensation system, the kit comprising:

microparticles modified by host molecules; and
a dilution solution for diluting the sample,
in which each of the host molecules binds specifically to the glycoprotein,
the dilution solution comprises a blocking agent and a buffering agent,
the pH value of the dilution solution is higher than the isoelectric point of the glycoprotein,
the concentration of the blocking agent is lower than a concentration at which the non-specific adsorption between the host molecules is inhibited in an environment where a light-induced force does not act on the host molecules, and
the salt concentration in the dilution solution is a concentration at which the microparticles modified by the host molecules cannot be precipitated by salting out.

[2] In the above [1], it is preferable that the dilution solution is neutral.

[3] In the above [1] or [2], it is preferable that the salt concentration in the dilution solution is a concentration at which the microparticles modified by the host molecules cannot be precipitated by salting out, and at which the thickness of an electrical double layer in the microparticles is reduced.

[4] In any of the above [1] to [3], it is preferable that the microparticles further comprise an additive that increases an electrostatic repulsive force.

[5] In any of the above [1] to [4], it is preferable that the microparticles comprise two or more types of microparticles with different sizes.

[6] In any of the above [1] to [5], it is preferable that the host molecules comprise at least one selected from the group consisting of an antibody, a Fab fragment, a $F(ab')_2$ fragment, a Fv fragment, and a scFv.

[7] In any of the above [1] to [6], it is preferable that the blocking agent comprises at least one selected from the group consisting of albumin, gelatin, casein, and goat serum.

[8] In any of the above [1] to [7], it is preferable that the concentration of the blocking agent is 0.000001% by mass or more and less than 0.001% by mass with respect to the dilution solution.

[9] In any of the above [1] to [8], it is preferable that the buffering agent comprises at least one selected from the group consisting of a phosphate compound, trishydroxymethylaminomethane, HEPES, and MES.

[10] In any of the above [1] to [9], it is preferable that the kit further comprises a dispersing solution for dispersing the microparticles modified by the host molecules,

the dispersing solution comprises the blocking agent and the buffering agent, and
the concentration of the blocking agent is 0.00000001% by mass or more and 0.001% by mass or less with respect to the dispersing solution.

[11] In any of the above [1] to [10], it is preferable that the glycoprotein is a cancer marker protein.

[12] In the above [11], it is preferable that the cancer marker protein comprises at least one selected from the group consisting of a carcinoembryonic antigen (CEA) and a carbohydrate antigen (such as CA19-9).

[13] In any of the above [1] to [12], it is preferable that the sample is a sample that has been frozen after collection or has been refrigerated for a predetermined period of time and then stored frozen, and
the glycoprotein contained in the sample aggregates to form a multimer or a nanoparticle.

[14] In any of the above [1] to [13], it is preferable that the kit further comprises a microchannel chip.

[15] The other kit according to the present invention is a kit for detecting a glycoprotein contained in a sample using an optical condensation system, the kit comprising:

microparticles modified by host molecules;
a dilution solution for diluting the sample; and
a dispersing solution for dispersing the microparticles modified by the host molecules,
in which each of the host molecules binds specifically to the glycoprotein,
the dilution solution comprises a buffering agent,
the dispersing solution comprises a blocking agent and the buffering agent,
the pH value of the dilution solution is higher than the isoelectric point of the glycoprotein,
the concentration of the blocking agent is lower than a concentration at which the non-specific adsorption between the host molecules is inhibited in an environment where a light-induced force does not act on the host molecules, and
the salt concentration in the dilution solution is a concentration at which the microparticles modified by the host molecules cannot be precipitated by salting out.

ADVANTAGEOUS EFFECTS OF INVENTION

According to the above, there can be provided a kit for detecting a minute amount of a glycoprotein contained in a sample rapidly and with high sensitivity using an optical condensation system.

BRIEF DESCRIPTION OF DRAWINGS

Fig. 1 is a schematic diagram describing the mechanism of an optical condensation system.

Fig. 2 is a schematic cross-sectional view of a microchannel chip 90 along the II-II line in Fig. 1.

Fig. 3 describes the aggregation mechanism of latex beads (microparticles modified by host molecules) when a measurement sample is irradiated with a laser beam.

Fig. 4 is a conceptual diagram for describing the detection principle for a certain glycoprotein.

Fig. 5 is a conceptual diagram for describing another detection principle for a glycoprotein.

Fig. 6 describes the aggregation mechanism of latex beads under defocus conditions.

Fig. 7 is graphs showing the correlation between the proportion of multilayer portions (vertical axis) and the concentration of a glycoprotein in a measurement sample (horizontal axis) when the measurement sample is measured using the optical condensation system.

Fig. 8 is graphs showing the correlation between the overall assembled area, the assembled area of multilayer portions, or the proportion of multilayer portions (vertical axis) and the concentration of a glycoprotein in a measurement sample (horizontal axis) when the measurement sample is measured using the optical condensation system.

Fig. 9 is graphs showing the correlation between the overall assembled area, the assembled area of multilayer portions, or the proportion of multilayer portions (vertical axis) and the concentration of a glycoprotein in a measurement sample (horizontal axis) when the measurement sample is measured using the optical condensation system.

Fig. 10 is photographs showing the vicinity of a laser spot when a measurement sample is measured using the optical condensation system.

Fig. 11A is graphs showing the correlation between the overall assembled area, the assembled area of multilayer portions, or the proportion of multilayer portions (vertical axis) and the concentration of a glycoprotein in a measurement sample (horizontal axis) when the measurement sample is measured using the optical condensation system in the case where the BSA concentration in an antibody B-modified bead dispersing solution is $1.65 \times 10^{-8}\%$ and the BSA concentration in an antibody A-modified bead dispersing solution is $2.16 \times 10^{-4}\%$.

Fig. 11B is graphs showing the correlation between the overall assembled area, the assembled area of multilayer portions, or the proportion of multilayer portions (vertical axis) and the concentration of a glycoprotein in a measurement sample (horizontal axis) when the measurement sample is measured using the optical condensation system in the case where the BSA concentration in an antibody B-modified bead dispersing solution and in an antibody A-modified bead dispersing solution is $3.6 \times 10^{-5}\%$.

Fig. 12 is graphs showing the correlation between the assembled area of multilayer portions (vertical axis) and the concentration of a glycoprotein in a measurement sample (horizontal axis) when the measurement sample is measured using the optical condensation system.

Fig. 13 is a schematic diagram describing the state of a glycoprotein in a measurement sample when the sample is stored frozen.

Fig. 14 is graphs showing the correlation between the light intensity (vertical axis) and the concentration of a glycoprotein in a measurement sample (horizontal axis) when the measurement sample is measured using the ELISA method.

Fig. 15 is a graph (left) showing the assembled area of multilayer portions (vertical axis) when a measurement sample (plasma sample, horizontal axis) is measured using the optical condensation system, and a graph (right) showing the concentration of a glycoprotein (vertical axis) when a measurement sample (plasma sample, horizontal axis) is measured using the ELISA method.

Fig. 16 is graphs showing the assembled area of multilayer portions (vertical axis) when a measurement sample (plasma sample, horizontal axis) is measured using the optical condensation system.

Fig. 17 is a graph showing the relationship between the distance from the particle surface and the electric potential.

Fig. 18 is graphs showing the pH-dependence and antibody modification amount-dependence of the correlation between the overall assembled area, the assembled area of multilayer portions, or the proportion of multilayer portions (vertical axis) and the concentration of a glycoprotein in a measurement sample (horizontal axis) when the measurement sample is measured using fluorescent beads as the probe particles and the optical condensation system.

Fig. 19 is graphs showing the laser power-dependence of the correlation between the overall assembled area, the assembled area of multilayer portions, or the proportion of multilayer portions (vertical axis) and the concentration of a glycoprotein in a measurement sample (horizontal axis) when the measurement sample is measured using fluorescent beads as the probe particles and the optical condensation system.

Fig. 20 is graphs showing the overall assembled area, the assembled area of multilayer portions, or the assembled area of multilayer portions (vertical axis) when a measurement sample (plasma sample, horizontal axis) is measured using fluorescent beads as the probe particles and the optical condensation system (laser irradiation time 5 min).

Fig. 21 is graphs showing the overall assembled area, the assembled area of multilayer portions, or the assembled area of multilayer portions (vertical axis) when a measurement sample (plasma sample, horizontal axis) is measured using fluorescent beads as the probe particles and the optical condensation system (laser irradiation time 4 min).

Fig. 22 is graphs showing the correlation between the overall assembled area, the assembled area of multilayer portions, or the proportion of multilayer portions (vertical axis) and the concentration of a glycoprotein and sodium chloride concentration in a measurement sample (horizontal axis) when the measurement sample is measured using fluorescent beads as the probe particles, adjusting the salt concentration in a dilution solution, and using the optical condensation system.

Fig. 23 is a schematic diagram for describing the blocking method in a microchannel.

Fig. 24 is graphs showing the correlation between the overall assembled area, the assembled area of multilayer portions, or the proportion of multilayer portions (vertical axis) and the concentration of a glycoprotein in a measurement sample (horizontal axis) when the measurement sample is measured using fluorescent beads as the probe particles and the optical condensation system in which a microchannel is blocked.

Fig. 25 is graphs showing the correlation between the overall assembled area, the assembled area of multilayer portions, or the proportion of multilayer portions (vertical axis) and the concentration of a glycoprotein in a measurement sample (horizontal axis) when the measurement sample is measured using two types of fluorescent beads with diameters of 2 $\mu$m and 1 $\mu$m mixed at a volume ratio of 1:0.05 as the probe particles and the optical condensation system.

Fig. 26 is a conceptual diagram of the mechanism of accumulated structure stabilization when measurement samples prepared by varying the mixing ratio of two types of fluorescent beads with different sizes are measured using the optical condensation system, and graphs showing the correlation between the proportion of multilayer portions (vertical axis) and the concentration of a glycoprotein in the measurement samples (horizontal axis).

DESCRIPTION OF EMBODIMENTS

Hereinafter, one embodiment of the present invention (hereinafter, sometimes referred to as "the present embodiment") will be described in detail with reference to the accompanying drawings. However, the present embodiment is not limited to this. Note that the same or equivalent portions in the drawings are marked with the same symbol and the description will not be repeated. In the present specification, the notation in the form of "A to Z" means the upper and lower limits of the range (that is, A or more and Z or less). In the case where no unit is indicated for A and a unit is indicated only for Z, the unit of A is the same as the unit of Z.

<<Kit for detecting glycoprotein contained in sample using optical condensation system>>

The kit according to the present embodiment is a kit for detecting a glycoprotein contained in a sample using an optical condensation system, the kit comprising:

microparticles modified by host molecules; and
a dilution solution for diluting the sample,
in which each of the host molecules binds specifically to the glycoprotein,
the dilution solution comprises a blocking agent and a buffering agent,
the pH value of the dilution solution is higher than the isoelectric point of the glycoprotein,
the concentration of the blocking agent is lower than a concentration at which the non-specific adsorption between the host molecules is inhibited in an environment where a light-induced force does not act on the host molecules, and
the salt concentration in the dilution solution is a concentration at which the microparticles modified by the host molecules cannot be precipitated by salting out.

<Optical condensation system>

In the present embodiment, the "optical condensation system" means a technology that utilizes the effect of causing a convection flow in a liquid due to the action of light on a substance, such as light-induced force and photo-induced convection flow, and the photothermal effect to cause agglomeration or accumulation of the target substance in a predetermined region. In one aspect of the present embodiment, in the case where the target substance is present in a liquid, the optical condensation system can also be interpreted as being able to condense the target substance in a predetermined region of the liquid (the region irradiated with light). In the present embodiment, the target substance may be the glycoprotein, which is an analyte, the host molecules, and the microparticles. Hereinafter, detailed description will be given.

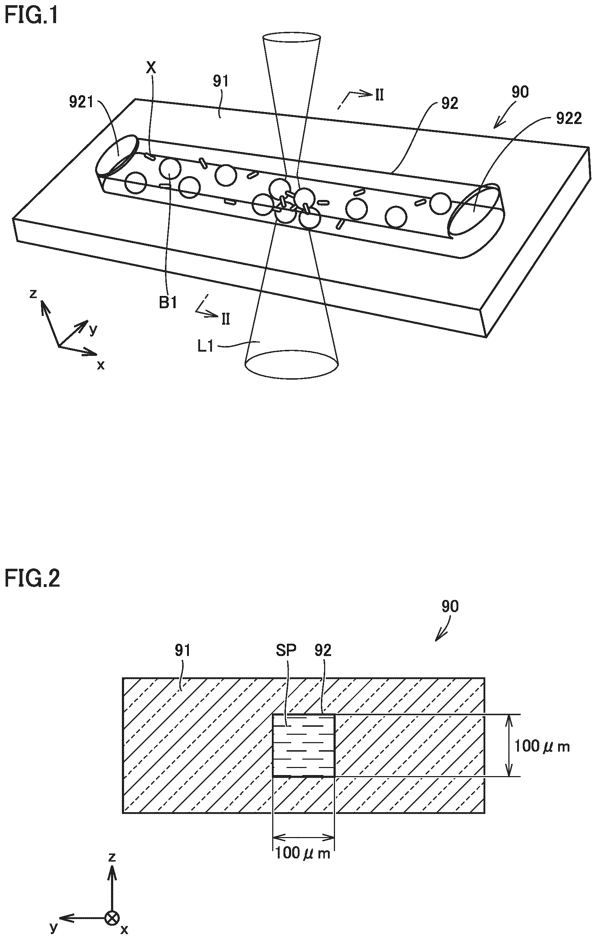

Fig. 1 is a schematic diagram describing the mechanism of the optical condensation system. Fig. 2 is a schematic cross-sectional view of a microchannel chip 90 along the II-II line in Fig. 1. In Fig. 1, a measurement sample SP is introduced into a microchannel 92 formed in a substrate 91 of the microchannel chip 90. In the example shown in Fig. 1, the measurement sample SP is introduced from an inlet 921, passes through the microchannel 92, and is discharged from an outlet 922. Note that the distribution direction of the sample SP in the microchannel 92 is defined as the x-direction. The measurement sample SP is, for example, a solution in which the sample is diluted with the dilution solution. The measurement sample SP contains microparticles modified by host molecules.

The measurement sample SP introduced into the microchannel 92 is irradiated with a laser beam L1 in a predetermined region. The laser beam L1 captures microparticles in the measurement sample SP by generating a light-induced force. Here, the "light-induced force" is used as a generic term for dissipative force, gradient force, and inter-object photo-induced force. The dissipative force is the force generated by the momentum of light given to a substance in a dissipative process such as light scattering or light absorption. The gradient force is the force that, when a substance with photo-induced polarization is placed in a non-uniform electromagnetic field, moves the substance to a point of stability in the electromagnetic potential. The inter-object photo-induced force is the sum of the force due to the longitudinal electric field resulting from induced polarization in a plurality of photo-excited substances and the force due to the transverse electric field (radiation field).

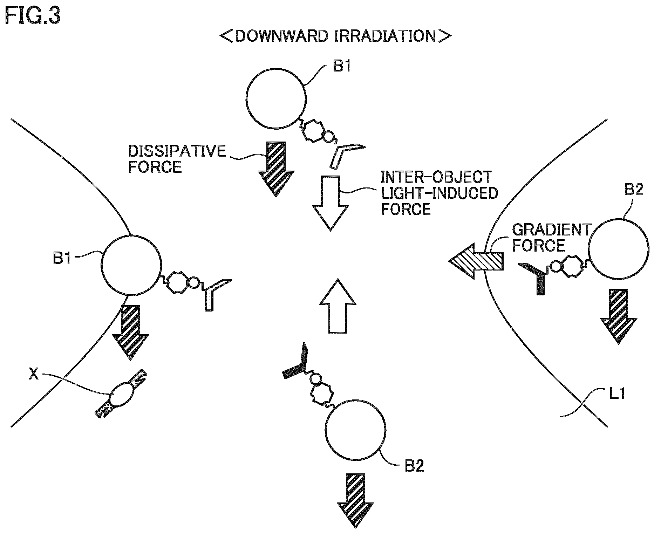

Fig. 3 describes the aggregation mechanism of latex beads (microparticles modified by host molecules) when the measurement sample is irradiated with the laser beam. After adjustment such that the beam waist of the laser beam L1 is positioned in the measurement sample SP, the microchannel chip 90 is irradiated with the laser beam L1, which causes beads B1 and B2 to gather in the vicinity of the beam waist due to a light-induced force (more specifically, inter-object photo-induced force and gradient force). This causes the density of the beads B1 and B2 in the vicinity of the beam waist to be locally higher than the density of the beads B1 and B2 at other positions (sufficiently remote from the beam waist).

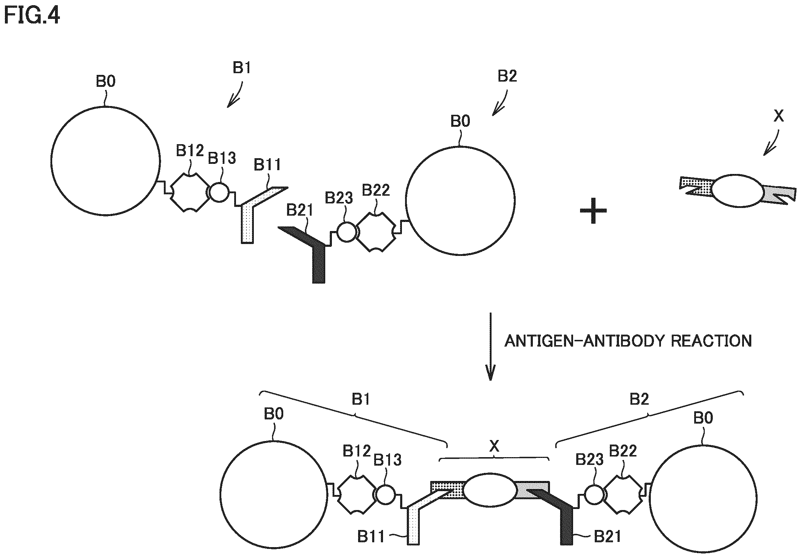

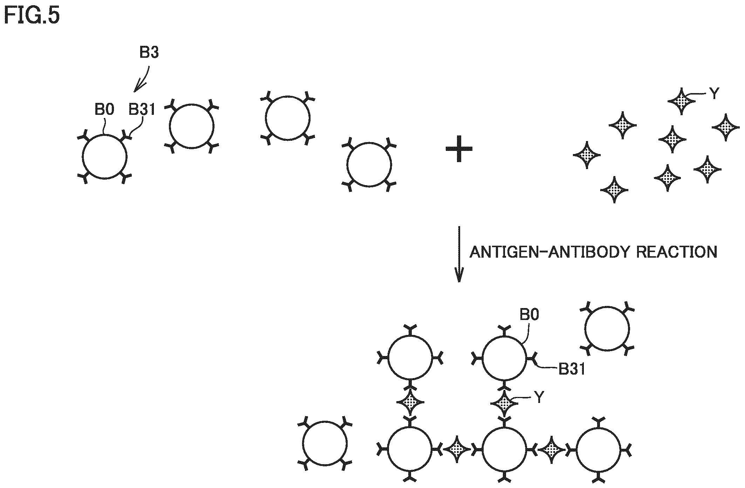

In the case where an analyte X (glycoprotein) is present around the beam waist, antigen-antibody reactions occur between a first antibody B11 (host molecule) modifying the surface of the bead B1 and the analyte X, and between a second antibody B21 (host molecule) modifying the surface of the bead B2 and the analyte X, causing the bead B1 and the bead B2 to bind to each other via the analyte X (see Fig. 4 and Fig. 5). The antigen-antibody reactions will be described later. By newly introducing the measurement sample SP from the inlet 921, a new analyte X is successively supplied around the beam waist. Therefore, the antigen-antibody reactions easily occur compared to those in a static liquid. The antigen-antibody reactions are repeated to form aggregates of the beads B1 and B2.

As the size of the aggregates of the beads B1 and B2 increases, the probability that the analyte X present around the aggregates encounters the aggregates increases, thus increasing the frequency of the antigen-antibody reactions. In other words, it is possible to realize "photo-induced acceleration" in which the aggregation of the beads B1 and B2 is accelerated by the irradiation with the laser beam L1 to the measurement sample SP. As a result, aggregates in which the beads B1 and B2 are aggregated to a high density are formed in a short time. Then, by optically detecting the aggregates formed, it can be rapidly determined that the measurement sample SP contains the analyte X.

In addition to the inter-object photo-induced force and the gradient force, the dissipative force acts on the beads B1 and B2 in the same direction as the irradiation direction of the laser beam L1. In the case of downward irradiation, the beads B1 and B2 are pressed against the bottom of the microchannel 92 by the dissipative force acting from above to below (Fig. 3).

Fig. 4 is a conceptual diagram for describing the detection principle for a certain glycoprotein. In the present embodiment, the glycoprotein, which is the analyte, is detected using the so-called latex aggregation method. In more detail, in the example shown in Fig. 4, two types of beads, B1 and B2, are prepared.

Each of the beads B1 and B2 contains a common bead body B0. The bead body B0 may be a microparticle, as will be described later. The bead body B0 is, for example, a resin bead (latex bead) composed of polystyrene. The bead body B0 has a size on the order of micrometers (typically a size with a diameter of about 1 $\mu$m to 5 $\mu$m), similar to general latex beads. The material of the bead body B0 may be other resins such as acrylic, polyolefin, polyethylene, and polypropylene.

In the bead B1, the bead body B0 is modified by the first antibody B11 (first host molecule). For the modification by the first antibody B11, avidin B12 and biotin B13 are used. The avidin B12 is fixed to the surface of the bead body B0 by the interaction between the avidin B12 and the bead body B0. The biotin B13 binds to the first antibody B11, thereby labeling the first antibody B11. The first antibody B11 modifies the surface of the bead body B0 due to strong affinity between the avidin B12 and the biotin B13.

In the bead B2, the bead body B0 is modified by the second antibody B21 (second host molecule). Similar to the first antibody B11, the second antibody B21 also modifies the surface of the bead body B0 by avidin B22 and biotin B23.

In the present embodiment, the analyte X in the example shown in Fig. 4 is a glycoprotein. Specifically, cancer marker proteins such as CEA and CA19-9 can be used as the analyte X. The analyte X has a site to which the host molecule binds specifically. In the case where the host molecule is an antibody, the site is called "epitope", "antigenic determinant", or "antibody recognition site". The analyte X may be a glycoprotein having a plurality of epitopes. Examples of such a glycoprotein include CEACAM-5 in [Examples] described later.

The analyte X undergoes an antigen-antibody reaction with the first antibody B11 and also undergoes an antigen-antibody reaction with the second antibody B21. Therefore, in the presence of the analyte X, the bead B1 and the bead B2 bind to each other via the analyte X. Fig. 4 shows an example in which the bead B1 is modified by only one first antibody B11. However, the actual bead B1 is modified by a larger number of first antibodies B11. The same is true for the bead B2. Therefore, when a plurality of beads B1 and B2 are introduced into a sample containing the analyte X, aggregates of the beads B1 and B2 are formed by aggregation of the plurality of beads B1 and B2 through antigen-antibody reactions.

Fig. 5 is a conceptual diagram for describing another detection principle for a glycoprotein. Depending on the type of glycoprotein that is the analyte, two types of beads are not always necessary. In the example shown in Fig. 5, only one type of bead B3 is prepared. The bead B3 includes the bead body B0 and an antibody B31 modifying the bead body B0.

The analyte Y in this example is also a glycoprotein, and specifically, aggregates of CEACAM-5 in [Examples] described later, for example. In the case where a plurality of beads B3 and the analyte Y are combined as well, the plurality of beads B3 aggregate due to antigen-antibody reactions with the analyte Y, and aggregates of the beads B3 are formed.

In the present embodiment, aggregates of the beads are formed utilizing the principle as described above, and the analyte (that is, glycoprotein) can be detected and quantified by observing such aggregates.

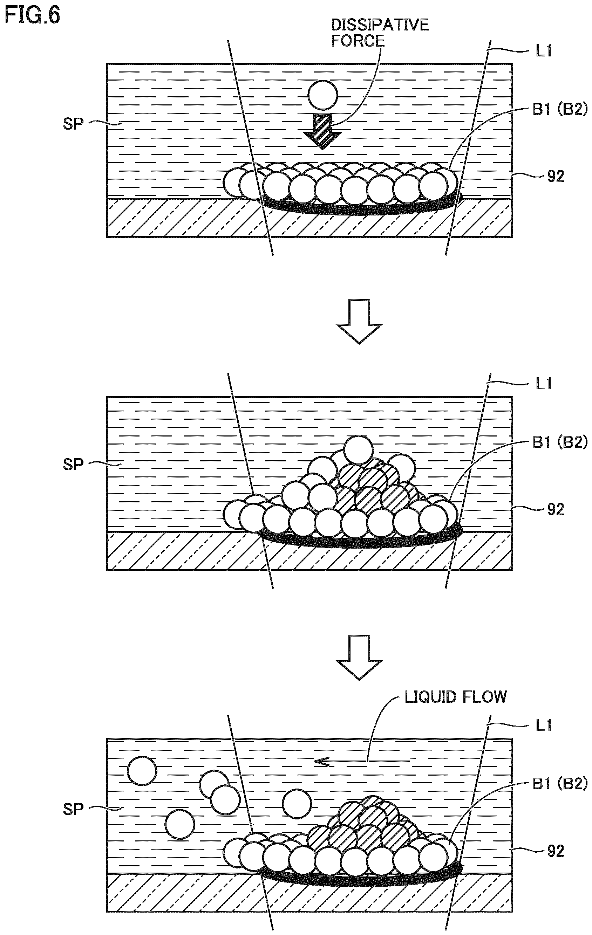

Fig. 6 describes the aggregation mechanism of latex beads (beads B1 and B2) under defocus conditions. In more detail, the defocus conditions are conditions when the beam waist of the laser beam L1 is positioned behind the microchannel 92 in the irradiation direction of the laser beam L1. In Fig. 6, the laser beam L1 at the bottom of the microchannel 92 (laser spot) is shown as a black circle.

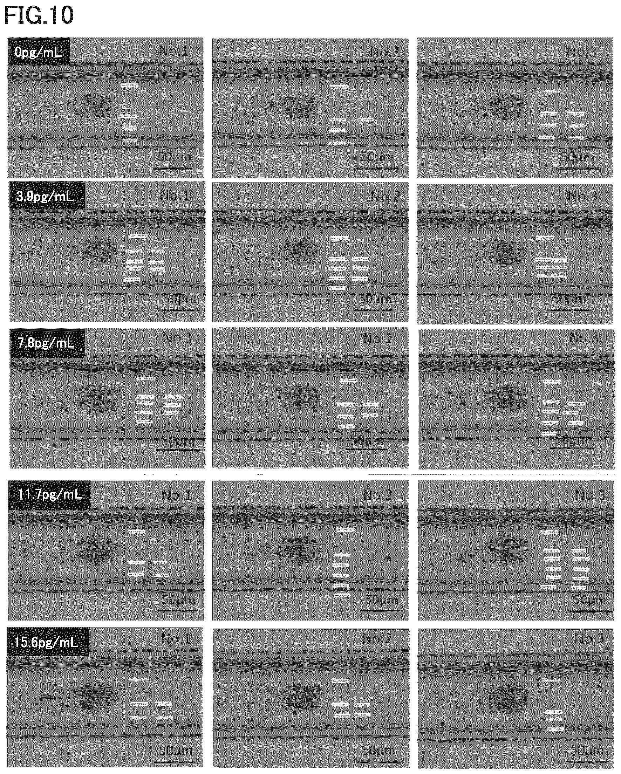

The beads B1 and B2 irradiated with the laser beam L1 are pressed against the bottom of the microchannel 92 by a light-induced force (in particular, dissipative force) of the laser beam L1. This results in the beads B1 and B2 lining up in the form of a monolayer on the bottom of the microchannel 92 (see the upper diagram in Fig. 6). Other beads B1 and B2 are further pressed on top of that monolayer, thereby forming a multilayer structure of the beads B1 and B2 (see the middle figure in Fig. 6). However, the beads B1 and B2 that do not bind to each other via the analyte X are pushed away in the distribution direction of the measurement sample SP (see the lower diagram in Fig. 6). As a result of such a "washing effect", so to speak, a region remains in which a multilayer structure of the beads B1 and B2 binding to each other via the analyte X is formed. For example, the dark-colored region shown in Fig. 10 is considered to be a region where a multilayer structure of the beads B1 and B2 is maintained as a result of antigen-antibody reactions.

As described above, the dark-colored region is a region that depends on the amount of the beads B1 and B2 binding to each other via the analyte X. In contrast, the light-colored region is basically a region defined depending on the size of the laser spot, regardless of whether the beads B1 and B2 bind to each other via the analyte X or not. Accordingly, under the defocus conditions, the concentration of the analyte X can be quantified with high accuracy by using the area of the dark-colored region as the evaluation target.

In the present embodiment, the area of the dark-colored region is normalized for convenience. Specifically, the proportion of the area of the dark-colored region to the area of the light-colored region (the entire area inside the light-colored outline in the image, hereinafter sometimes simply referred to as "the entire area") is defined as the "proportion of multilayer portions".

$$\text{proportion of multilayer portions} = \text{area of dark-colored region} / \text{entire area}$$

Here, the area of the light-colored region and the area of the dark-colored region are both determined by automatically performing area measurement using the image analysis software NIS Elements manufactured by Nikon Corporation.

<Sample>

In the present embodiment, the "sample" means a substance that contains the analyte or a substance that may contain the analyte. In the present embodiment, the analyte includes a glycoprotein. That is, the sample may contain a glycoprotein. The sample may be, for example, a biological sample from animals (for example, human, cow, horse, pig, goat, chicken, rat, mouse, and others). The biological sample may include, for example, blood, tissues, cells, secretions, body fluids, and others. Note that the "sample" may include diluted products or isolated products (such as serum and plasma) thereof. "Liquid sample" is a liquid containing the sample.

In one aspect of the present embodiment, it is preferable that the sample is a sample that has been frozen after collection or has been refrigerated for a predetermined period of time and then stored frozen. By doing so, the glycoprotein contained in the sample forms a multimer or a nanoparticle, and the glycoprotein can be efficiently detected or quantified even in the case of using one type of host molecules. The period of time for which the sample is stored refrigerated before freezing may be, for example, 1 day or longer and 10 days or shorter.

(Glycoprotein)

In the present embodiment, the "glycoprotein" means a substance in which a sugar chain binds to part of an amino acid residue that constitutes a protein. Although the glycoprotein is not particularly limited, examples thereof include CEA, CA19-9, CD99, AFP, immunoglobulin, collagen, mucin, follicle-stimulating hormone, erythropoietin, transferrin, and lectin. In one aspect of the present embodiment, it is preferable that the glycoprotein is a cancer marker protein. It is preferable that the cancer marker protein include at least one selected from the group consisting of CEA, CD99, AFP, and CA19-9.

\<Microparticles\>

In the present embodiment, the "microparticles" mean a substance having a size on the order of nanometers to micrometers. In the present embodiment, the microparticles are modified by the host molecules, which will be described later. The shape of the microparticles is not limited to a spherical shape, and may be ellipsoidal, rod-shaped, or others. In the case where the microparticles are ellipsoidal, it is sufficient if at least one of the lengths of the ellipsoid in the major axis direction and in the minor axis direction is in the range on the order of nanometers to micrometers. In the case where the microparticles are rod-shaped, it is sufficient if at least one of the width and length of the rod is in the range on the order of nanometers to micrometers.

Examples of the microparticles include metallic nanoparticles, metallic nanoparticle assemblies, metallic nanoparticle accumulated structures, semiconductor nanoparticles, organic nanoparticles, resin beads, magnetic beads, and particulate matters (PM). The "metallic nanoparticles" are metallic particles having a size on the order of nanometers. The "metallic nanoparticle assemblies" are assemblies formed by aggregation of a plurality of metallic nanoparticles. The "metallic nanoparticle accumulated structures" are, for example, structures in which a plurality of metallic nanoparticles are fixed to the surface of beads via interactive sites and arranged with gaps between each other and at intervals of less than or equal to the diameter of the metallic nanoparticles. The "semiconductor nanoparticles" are semiconductor particles having a size on the order of nanometers. The "organic nanoparticles" are particles composed of an organic compound having a size on the order of nanometers. The "resin beads" are particles composed of a resin having a size on the order of nanometers to micrometers. Examples of the resin beads include resin beads composed of polystyrene. The "magnetic beads" are magnetically charged particles (polymer microparticles with a magnetic material dispersed or embedded inside) having a size on the order of nanometers to micrometers. The "PM" is particulate matters having a size on the order of micrometers.

In one aspect of the present embodiment, the microparticles may further contain an additive that increases an electrostatic repulsive force. Here, examples of the "additive that increases an electrostatic repulsive force" include a fluorescent dye and a surface-modifying reagent. Examples of the functional group imparted by the surface-modifying reagent include a carboxyl group and an amino group. In the case where the microparticles are resin beads, the resin beads may contain a fluorescent dye. As shown in Examples described later, resin beads containing a fluorescent dye (hereinafter, sometimes referred to as "fluorescent beads") have a strong negative surface potential (zeta potential). Therefore, non-specific adsorption between host molecules (for example, antibodies) modifying the microparticles tends to be inhibited, which in turn tends to improve measurement accuracy. In the case where another "additive that increases an electrostatic repulsive force" other than fluorescent beads is used as well, the present inventors believe that the same mechanism will work.

In the present embodiment, "on the order of nanometers" includes the range from 1 nm to 1000 nm (=1 $\mu$m). "On the order of micrometers" includes the range from 1 $\mu$m to 1000 $\mu$m (=1 mm). Accordingly, "on the order of nanometers to micrometers" includes the range from 1 nm to 1000 $\mu$m. The term "on the order of nanometers to micrometers" may typically mean the range from several nm to several hundred $\mu$m, preferably mean the range from 100 nm to 100 $\mu$m, and more preferably mean the range from 1 $\mu$m to several ten $\mu$m.

In one aspect of the present embodiment, the size (for example, diameter, major diameter, and minor diameter) of the microparticles is preferably 100 nm or more and 100 $\mu$m or less, more preferably 1 $\mu$m or more and 10 $\mu$m or less. In the case where the wavelength of a laser beam with which the microparticles are irradiated is 1064 nm, Mie scattering easily occurs by setting the size of the microparticles to the range as described above, which in turn tends to generate a strong light-induced force. The size of the microparticles can be measured by, for example, the dynamic light scattering method.

In one aspect of the present embodiment, the microparticles may contain two or more types of microparticles with different sizes, or may contain two types of microparticles with different sizes. For example, fluorescent beads with a size of 2 μm and fluorescent beads with a size of 1 μm may be mixed for use. Examples of the mixing ratio include the ratio between the number of 2 μm beads and 1 μm beads in the range of 1:8 to 1:0, with a preferred ratio of 1: 1 to 1:0.01. Such a range allows for less non-specific adsorption between beads, and also allows for formation of a multilayer structure that is stable in the optical condensation system.

(Host molecules)

In the present embodiment, the "host molecules" mean a substance that can bind specifically (even by specific attachment) to the analyte. Examples of the combination of host molecules and analyte include antigen and antibody, sugar chain and protein, lipid and protein, low molecular weight compound (ligand) and protein, protein and protein, and single-stranded DNA and single-stranded DNA. In the case where one of these two having specific affinity for each other is the analyte, the other can be used as the host molecules. That is, for example, in the case where an antigen is the analyte, an antibody can be used as the host molecules. On the contrary, in the case where an antibody is the analyte, an antigen can be used as the host molecules. Also, in DNA hybridization, the analyte is a target DNA and the host molecules are a probe DNA. In addition, anti-DNA antibodies that bind specifically to DNA (for example, anti-dsDNA antibodies that bind specifically to double-stranded DNA, anti-ssDNA antibodies that bind specifically to single-stranded DNA, and others) can also be used as the host molecules. Note that the antigen may include cancer marker proteins, allergens, microorganisms (such as bacteria and fungi), viruses, vesicles, and others. Also, by changing the type of antibody, the type of cancer marker protein, allergen, microorganism, or virus that can be detected can be changed as well.

The host molecules are fixed to (sometimes described as "modify") the surface of the microparticles by the interaction between the host molecules and the microparticles. The type of interaction used to fix the host molecules to the surface of the microparticles is determined depending on the type of microparticles (for example, the material of the microparticles). The interaction includes, for example, covalent bond, ionic bond, metallic bond, van der Waals force, electrostatic interaction, hydrophobic interaction, intermolecular force (for example, hydrogen bond), adsorption force, and others. There is no particular restriction on the method for fixing the host molecules to the surface of the microparticles, and any known method can be employed. In the case where the host molecules are fixed to the surface of the microparticles in a liquid, it is preferable to do so in a liquid in which the blocking agent described later is present from the viewpoint of inhibiting non-specific adsorption between the host molecules.

In the present embodiment, the host molecules bind specifically to the glycoprotein. Examples of the host molecules include an antibody, a Fab fragment, a F(ab')$_2$ fragment, a Fv fragment, and a scFv. In one aspect of the present embodiment, it is preferable that the host molecules include at least one selected from the group consisting of an antibody, a Fab fragment, a F(ab')$_2$ fragment, a Fv fragment, and a scFv. There is no particular restriction on the animal from which the antibody is derived, and examples thereof include mouse, sheep, goat, camel, rat, and rabbit. The Fab fragment and F(ab')$_2$ fragment, as well as the Fv fragment and scFv, can be produced by known methods (for example, method using enzymes and method using genetic engineering).

<Dilution solution>

In the present embodiment, the "dilution solution" means a liquid for diluting the sample. The dilution solution contains a blocking agent and a buffering agent. The solvent that constitutes the dilution solution is normally water such as ion-exchanged water and distilled water. There is no particular restriction on the method for producing the dilution solution, and it can be produced by a general method. Examples thereof include a method in which the blocking agent and the buffering agent are added to ion-exchanged water and dissolved to reach predetermined concentrations. The obtained dilution solution may then undergo a post-treatment such as filter sterilization.

The pH value of the dilution solution is higher than the isoelectric point of the glycoprotein. Also, it is preferable that the dilution solution is neutral. By doing so, non-specific adsorption between the microparticles modified by the host molecules can be inhibited. As a result, the accuracy and sensitivity of detection and quantification of a glycoprotein using the optical condensation system is improved. Specifically, the pH value of the dilution solution is preferably 4.8 or more and 9 or less, more preferably 6 or more and 8 or less, and still more preferably 6 or more and 7.2 or less. The pH value of the dilution solution can be measured with a pH meter generally used.

In the case where the glycoprotein is a carcinoembryonic antigen (isoelectric point: 4.7), the pH value of the dilution solution is preferably 4.7 or more and 7.0 or less, more preferably 6.3 or more and 6.8 or less.

In the present embodiment, the salt concentration in the dilution solution is a concentration at which the microparticles modified by the host molecules cannot be precipitated by salting out. By inhibiting salting out of the microparticles modified by the host molecules, the accuracy and sensitivity of detection and quantification of a glycoprotein using the optical condensation system is improved. The salt concentration of the dilution solution may be 0 ng/mL or more and 100 ng/mL or less, or may be 1 ng/mL or more and 10 ng/mL or less. Here, in the case where there are multiple types of salts contained in the dilution solution, the total concentration of each salt is defined as the "salt concentration in the dilution solution". The salt concentration can be presumed from, for example, where salting out due to optical condensation occurs for the beads, independent of the concentration of the analyte.

In one aspect of the present embodiment, it is preferable that the salt concentration in the dilution solution is a concentration at which the microparticles modified by the host molecules cannot be precipitated by salting out, and at which the thickness of an electrical double layer in the microparticles is reduced. By doing so, the binding specificity of the host molecules to the glycoprotein can be further improved. Here, the "thickness of an electrical double layer in the microparticles" is expressed as the reciprocal number of a parameter indicating the degree of attenuation of the surface potential, called the Debye length. The Debye length is closely correlated with the surface potential and the zeta potential. Therefore, it is possible to estimate the Debye length by measuring the zeta potential of the microparticles, and in turn, it is possible to estimate the thickness of the electrical double layer. In the case where the microparticles are fluorescent beads, the salt concentration in the dilution solution may be 80 ng/mL or more to 100 ng/mL, or may be 90 ng/mL or more to 100 ng/mL.

In the present embodiment, examples of the salt that may be contained in the dilution solution include sodium chloride and potassium chloride.

(Blocking agent)

In the present embodiment, the blocking agent is a substance for inhibiting non-specific adsorption between the host molecules in the sample.

Examples of the blocking agent include albumin (such as bovine serum albumin (BSA)), gelatin, casein, and goat serum. In one aspect of the present embodiment, it is preferable that the blocking agent includes at least one selected from the group consisting of albumin, gelatin, casein, and goat serum. Note that it goes without saying that the substance used as the blocking agent is a different substance from the glycoprotein that is the analyte.

In the present embodiment, the concentration of the blocking agent is lower than a concentration at which the non-specific adsorption between the host molecules is inhibited in an environment where a light-induced force does not act on the host molecules. By doing so, non-specific adsorption between the microparticles modified by the host molecules can be inhibited. As a result, the accuracy and sensitivity of detection and quantification of a glycoprotein using the optical condensation system is improved. It is preferable that the concentration of the blocking agent is 0.000001% by mass or more and less than 0.001% by mass with respect to the dilution solution. The concentration of the blocking agent can be determined by, for example, the ELISA method using an anti-BSA antibody or the optical condensation system.

(Buffering agent)

In the present embodiment, the "buffering agent" means a substance that inhibits pH fluctuations in the target liquid (for example, in the dilution solution) due to changes in the environment in such a liquid. Examples of the changes in the environment in the liquid include changes in salt concentration, mixing with other liquids, and changes in temperature.

Examples of the buffering agent include a phosphate compound, trishydroxymethylaminomethane, HEPES (4-(2-hydroxyethyl)-1-piperazineethanesulfonic acid), and MES (2-(N-morpholino)ethanesulfonic acid). In one aspect of the present embodiment, it is preferable that the buffering agent includes at least one selected from the group consisting of a phosphate compound, trishydroxymethylaminomethane, HEPES, and MES. Examples of the phosphate compound include phosphoric acid, disodium hydrogenphosphate, and potassium dihydrogenphosphate. Although there is no particular restriction on the concentration of the buffering agent, for example, it may be 0.1 mM or more and 1000 mM or less, or may be 1 mM or more and 100 mM or less.

(Other components)

The dilution solution according to the present embodiment may further contain other components to the extent that the effects of the present invention are achieved.

<Dispersing solution>

It is preferable that the kit according to the present embodiment further includes a dispersing solution for dispersing the microparticles modified by the host molecules. It is preferable that the dispersing solution contains the blocking agent and the buffering agent. The blocking agent contained in the dispersing solution may be the same component as the blocking agent contained in the dilution solution, or it may be a different component. Specific examples of the blocking agent contained in the dispersing solution include those described above. The concentration of the blocking agent is preferably 0.00000001% by mass or more and 0.001% by mass or less with respect to the dispersing solution. Such a concentration range allows for inhibition of non-specific adsorption between the microparticles. There is no particular restriction on the method for producing the dispersing solution, and it can be produced by a general method. Examples thereof include a method in which the blocking agent and the buffering agent are added to ion-exchanged water and dissolved to reach predetermined concentrations. The obtained dispersing solution may then undergo a post-treatment such as filter sterilization.

The buffering agent contained in the dispersing solution may be the same component as the buffering agent contained in the dilution solution, or it may be a different component. Specific examples of the buffering agent contained in the dispersing solution include those described above. Although there is no particular restriction on the concentration of the buffering agent, for example, it may be 0.1 mM or more and 1000 mM or less, or may be 1 mM or more and 100 mM or less.

In one aspect of the present embodiment, the microparticles modified by the host molecules and the dispersing solution may be included in the kit separately. Alternatively, the microparticles modified by the host molecules may be included in the kit in the state of being pre-dispersed in the dispersing solution. That is, the dispersing solution may contain the microparticles modified by the host molecules.

<Microchannel chip>

It is preferable that the kit further includes a microchannel chip. Examples of the microchannel chip include the microchannel chip 90 shown in Fig. 1. Fig. 2 is a schematic cross-sectional view of the microchannel chip 90 along the II-II line in Fig. 1. As shown in Fig. 2, the cross-section of the microchannel 92 has a rectangular shape, for example. As an example, the width of the rectangle (channel width, length in the y direction) is 100 $\mu$m, and the height of the rectangle (length in the z direction) is 100 $\mu$m.

It is preferable that the microchannel chip 90 is constituted by a material that is transparent to the laser beam L1. It is preferable that the material used for the microchannel chip 90 is a material that does not exhibit anisotropy to the laser beam L1, which is polarized light, such as glass or quartz, for example. In one aspect of the present embodiment, it is preferable that the surface of the microchannel 92 in the microchannel chip 90 is constituted by a material that does not non-specifically adsorb the analyte and the host molecules.

In one aspect of the present embodiment, it is preferable that the surface of the microchannel 92 in the microchannel chip 90 is blocked with a blocking agent. As the blocking agent, those listed above can be used. There is no particular restriction on the method of blocking, and any known method can be used. For example, the surface of the microchannel 92 can be blocked by the method described in Examples described later.

<Other configurations>

The kit according to the present embodiment may further include other configurations to the extent that the effects of the present invention are achieved. Examples of the other configurations include an instruction manual describing how to use the kit, microtubes, and a glycoprotein (positive control).

<<Method for using kit>>

The kit according to the present embodiment is used, for example, as follows. First, the dilution solution and microparticles are added to the sample to prepare the measurement sample. At this time, the dilution solution may be added to the sample before adding the microparticles, or the microparticles may be added to the sample before adding the dilution solution. Alternatively, the dilution solution and microparticles may be added to the sample at the same time. Here, the "measurement sample" means a sample immediately before being subjected to measurement using the optical condensation system.

Next, the prepared measurement sample is measured using the optical condensation system to detect or quantify the glycoprotein contained in the measurement sample. Examples of the measurement conditions at this time include the conditions described in [Examples] described later.

In the above, details of the kit according to the present embodiment have been described. Although it has conventionally been expected to use an optical condensation system to detect and quantify a minute amount of the analyte, how to specifically prepare the measurement sample and subject it to measurement has not been specifically examined. In the present embodiment, by making the dilution solution used to dilute the sample have the configuration as described above, it is possible to detect a minute amount of a glycoprotein contained in the sample rapidly and with high sensitivity using an optical condensation system. More specifically, a 10- to 100-fold improvement in sensitivity and a 60-fold increase in speed over the ELISA method conventionally used are achieved. Such a rapid and highly sensitive detection technique using an optical condensation system is expected to contribute to realization of ultra-early diagnosis of cancer.

<<Another embodiment>>

Another kit according to the present embodiment is a kit for detecting a glycoprotein contained in a sample using an optical condensation system, the kit comprising:

microparticles modified by host molecules;
a dilution solution for diluting the sample; and
a dispersing solution for dispersing the microparticles modified by the host molecules,
in which each of the host molecules binds specifically to the glycoprotein,
the dilution solution comprises a buffering agent,
the dispersing solution comprises a blocking agent and the buffering agent,
the pH value of the dilution solution is higher than the isoelectric point of the glycoprotein,
the concentration of the blocking agent is lower than a concentration at which the non-specific adsorption between the host molecules is inhibited in an environment where a light-induced force does not act on the host molecules, and
the salt concentration in the dilution solution is a concentration at which the microparticles modified by the host molecules cannot be precipitated by salting out. Examples

Hereinafter, Examples according to the present invention will be described, but the present invention is not limited to these. Note that "CEACAM-5" may be simply referred to as "CEA" in the following.

<<1. Fabrication of beads modified by antibodies>>

<Preparation of reagents>

In order to fabricate beads modified by antibodies (microparticles modified by host molecules), the following reagents were prepared.

(Beads (microparticles))

Streptavidin Coated Microsphere (resin beads modified by streptavidin): 2 μm Plain (manufactured by Polysciences, Inc., Cat. No. 24160, material: polystyrene): The beads are suspended in a 0.02M sodium phosphate buffer at a concentration of 1.25%. The sodium phosphate buffer contains NaCl (8 mg/mL), bovine serum albumin (BSA) (10 mg/mL), sodium azide (0.1%), and glycerol (5%).

(Antibodies (host molecules))

The following two antibodies packaged together in Human CEACAM-5/CD66e DuoSet ELISA (manufactured by R&D Systems, Cat. No. DY4128, kit for ELISA) were used.
Biotinylated sheep anti-human CEACAM-5 antibody (antibody A): 36 μg/mL (dissolved in PBS containing 1% BSA, pH 7.2 to 7.4), the composition of PBS is 137 mM of NaCl, 2.7 mM of KCl, 8.1 mM of $Na_2HPO_4$, and 1.5 mM of $KH_2PO_4$.
Mouse anti-human CEACAM-5 antibody (antibody B): 1 mg/mL (dissolved in PBS, pH 7.2 to 7.4), biotinylated by the method described later for use.

(Buffer)

10 mM phosphate buffer (pH 6.7): prepared by diluting 10 times with distilled water (DDW) a 0.1 mol/L phosphate buffer pH 6.4 (manufactured by FUJIFILM Wako Pure Chemical Corporation, catalog No. 164-27135).
10 mM phosphate buffer (pH 6.3): prepared by diluting 10 times with DDW a 0.1 mol/L phosphate buffer pH 6.0 <manufactured by FUJIFILM Wako Pure Chemical Corporation, catalog No. 167-27125>.
10 mM phosphate buffer (pH 7.2): prepared by diluting 10 times with DDW a 0.1 mol/L phosphate buffer pH 7.0 <manufactured by FUJIFII,M Wako Pure Chemical Corporation, catalog No. 168-27155>.
10 mM phosphate buffer (pH 7.0): prepared by diluting 10 times with DDW a 0.1 mol/L phosphate buffer pH 6.8 <manufactured by FUJIFILM Wako Pure Chemical Corporation, catalog No. 161-27145>.

<Biotinylation of antibody B>

The antibody B was biotinylated using EZ-Link™ Sulfo-NHS-LC-Biotin, No-Weigh™ Format <Thermo Scientific™/ Cat. No. A39257> according to the following procedure.

To 720 μL of the antibody B dissolved at 1 mg/mL using the 10 mM phosphate buffer pH 7.2, 13 μL of the biotinylation reagent was added, which was then allowed to stand still on ice for 2 hours to perform biotinylation. After standing still on ice, the excess biotinylation reagent was removed using a desalting column (Zeba Spin Desalting Columns 7K MWCO) equilibrated with the 10 mM phosphate buffer pH 6.7.

By the above procedure, the biotinylated antibody B was obtained. In the following descriptions, unless otherwise noted, references to "antibody B" mean the biotinylated antibody B.

<Antibody modification to beads>

From the suspension of streptavidin-modified beads, 18 μL were collected and transferred to microtubes. 90 μL of the 10 mM phosphate buffer (pH 6.7) was added to the microtubes. Thereafter, the microtubes were set in a centrifuge and centrifuged under the conditions of 10000 g × 5 min, and the beads were precipitated to the bottom of the microtubes. The supernatant was removed by 83.3% by volume from the microtubes, and then the same amount of the 10 mM phosphate buffer (pH 6.7) was added to the microtubes. By repeating this operation five times, the streptavidin-modified beads were washed.

The 10 mM phosphate buffer (pH 6.7) was added to the microtubes so as to dilute 3 times the suspension containing the streptavidin-modified beads after washing.

In the experiments of Study 1 to Study 4 described later, the antibody A and antibody B were each diluted with the 10 mM phosphate buffer (pH 6.7) such that the final concentrations were 20 μg/mL.

In the experiment of Study 5 described later, the antibody A was diluted with the 10 mM phosphate buffer (pH 6.7) such that the final concentration was 20 μg/mL. The antibody B was diluted with PBS containing 1% BSA (pH 7.2 to 7.4) such that the final concentration was 20 μg/mL.

The suspension containing the streptavidin-modified beads and the solution containing the antibody A or antibody B were mixed in equal amounts to form a mixed solution, which was then allowed to stand still under the conditions of 42°C for 1 hour.

Thereafter, the microtubes containing the mixed solution were set in a centrifuge and centrifuged under the conditions of 10000 g × 5 min, and the beads modified by the antibodies were precipitated to the bottom of the microtubes. The supernatant was removed by 83.3% by volume from the microtubes, and then the same amount of the 10 mM phosphate buffer (pH 6.7) was added to the microtubes. By repeating this operation three times, the beads modified by the antibodies were washed. Note that, in the second and third washings, the 10 mM phosphate buffer (pH 6.3) was used to perform the washing operation instead of the 10 mM phosphate buffer (pH 6.7). By the above operation, the beads modified by the antibodies were obtained. In view of the operation described above, it is considered that the suspension of the beads modified by the antibodies contains 7.89 ng/mL of NaCl and 23.7 ng/mL of KCl.

<<2. Study of conditions for detecting glycoprotein using optical condensation system>>

<Study 1: pH-dependence of liquid sample>

(Preparation of liquid sample)

Recombinant Human CEACAM-5 (glycoprotein) (concentration 100 ng/mL) packaged together in Human CEACAM-5/CD66e DuoSet ELISA (manufactured by R&D Systems, Cat. No. DY4128, kit for ELISA) was diluted with the dilution solution described later to final concentrations of 7.8 pg/mL, 15.6 pg/mL, and 31.2 pg/mL, thereby preparing three standard samples. For the dilution solution, three different phosphate buffers with different pH values were used (pH: 7.2, 7.0, and 6.7). The phosphate buffers were prepared by diluting commercially available phosphate buffers (phosphate buffers described above manufactured by FUJIFILM Wako Pure Chemical Corporation) 10 times with DDW. Also, as standard samples with a CEACAM-5 concentration of 0 pg/mL, the three dilution solutions described above were used as they were. That is, four standard samples with different CEACAM-5 concentrations were prepared for each phosphate buffer, which thus means that twelve standard samples with different pH values and CEACAM-5 concentrations were prepared.

20 μL of each of the twelve standard samples was transferred to microtubes, to which 20 μL of the suspension of beads modified by the antibody A in "1. Fabrication of beads modified by antibodies" described above was added and mixed well. By the above procedure, twelve measurement samples (pH 6.7 to 7.2, CEACAM-5 concentrations of 0 to 15.6 pg/mL) were prepared.

(Detection by optical condensation system)

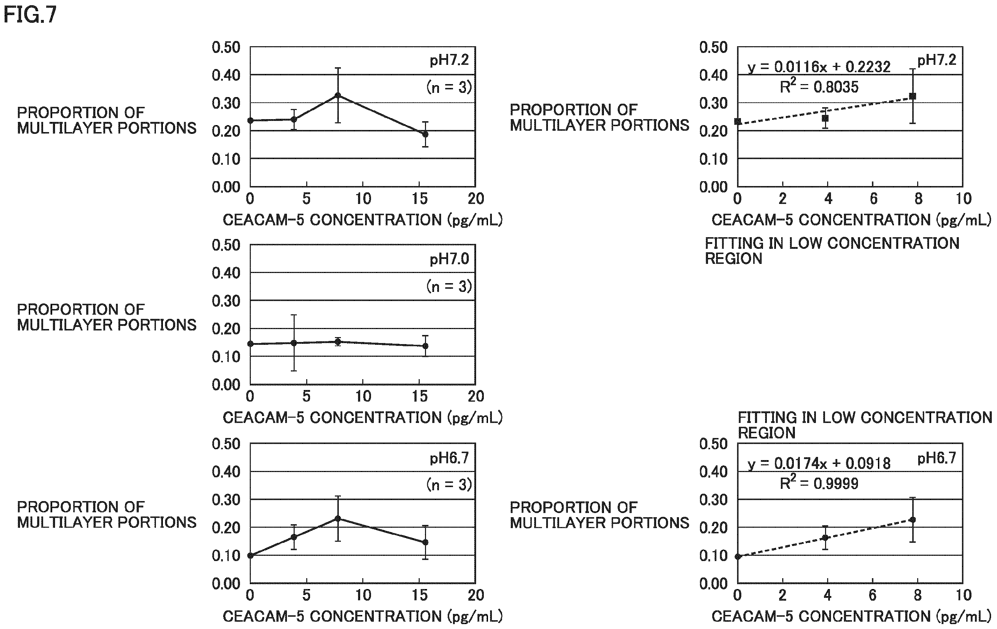

The prepared measurement samples were injected into the microchannel of the microchannel chip in the optical condensation system, and the measurement samples were irradiated with light under the following conditions. Thereafter, the assembled area of beads, the assembled area of multilayer portions, and the proportion of multilayer portions (hereinafter, "multilayer proportion") in the measurement field of view were determined by the method described above. The results are shown in Fig. 7. In Fig. 7, the vertical axis indicates the proportion of multilayer portions, and the horizontal axis indicates the concentration of CEACAM-5 in the measurement sample.

Conditions for irradiation with light

Microchannel: 100 $\mu$m $\times$ 100 $\mu$m
Syringe: inner diameter 2.3 mm / volume 0.25 mL
Flow rate: 250 $\mu$L syringe setting, 0.05 $\mu$L/min
Laser intensity: 265 mW irradiation for 5 min
Position of beam waist of x40 lens: 45 $\mu$m below the bottom of channel

The results in Fig. 7 show that a smaller pH value can reduce the proportion of multilayer portions at a CEACAM-5 concentration of 0 pg/mL. The reason for such results is thought to be that, in the case where the pH value in the measurement sample was 6.7, the positive charge due to hydrogen ions was imparted and electrostatic repulsion inhibited non-specific adsorption between the antibody-modified beads. Note that the pH value of the dilution solution was found to be higher than the isoelectric point of CEACAM-5 (4.7).

<Study 2: Study of beam waist position>

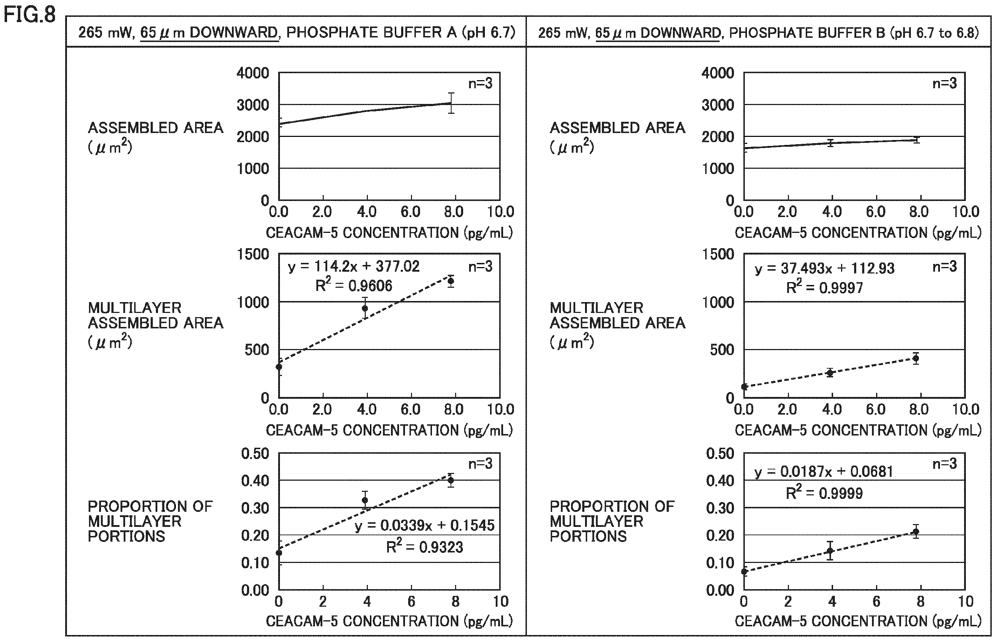

An experiment was performed by the same method as in Study 1, except that the following two phosphate buffers A or B were used as the dilution solution and the position of beam waist at the time of measurement was set to 65 $\mu$m below the bottom of the channel, and the assembled area of beads, the assembled area of multilayer portions, and the proportion of multilayer portions in the measurement field of view were determined by the predetermined method. The results are shown in Fig. 8. In Fig. 8, the vertical axis indicates the assembled area of beads, the assembled area of multilayer portions, or the proportion of multilayer portions, and the horizontal axis indicates the concentration of CEACAM-5 in the liquid sample.

Dilution solutions used

Phosphate buffer A (pH 6.7, not containing NaCl and KCl)
Phosphate buffer B (pH 6.7 to 6.8, not containing NaCl and KCl)

The results in Fig. 8 show that setting the position of beam waist at the time of measurement to 65 $\mu$m below the bottom of the channel improves sensitivity and measurement accuracy compared to when the position of beam waist is 45 $\mu$m below the bottom of the channel (see Fig. 7).

<Study 3: Blocking agent concentration-dependence>

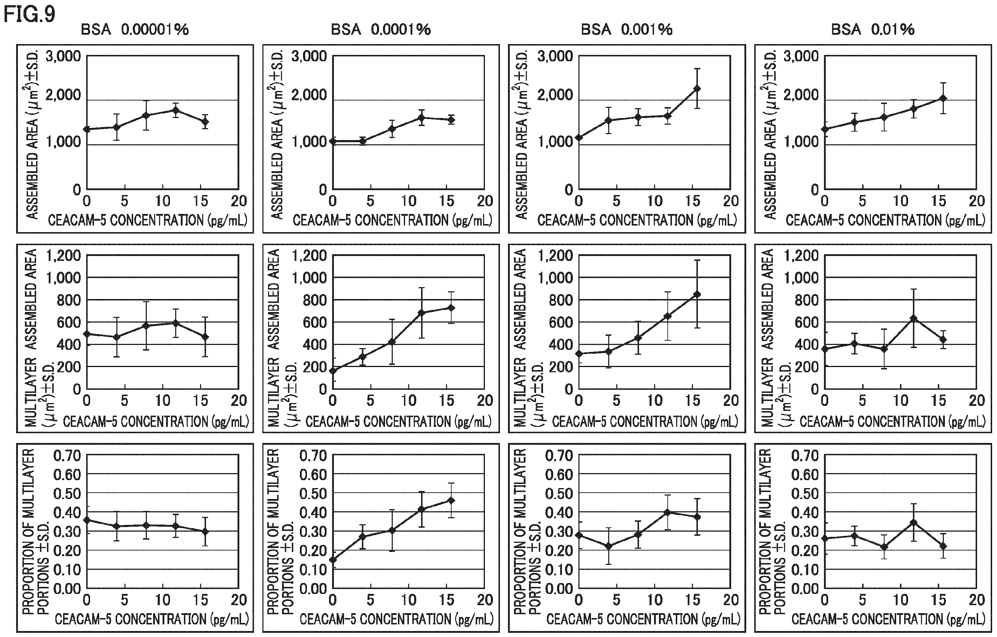

An experiment was performed by the same method as in Study 1, except that the following four phosphate buffers with different BSA concentrations were used as the dilution solution, 10 $\mu$L each of the suspension of beads modified by the antibody A and the suspension of beads modified by the antibody B were used as the suspension of beads, and the flow rate was changed to 0.1 $\mu$m/min, and the assembled area of beads, the assembled area of multilayer portions, and the proportion of multilayer portions in the measurement field of view were determined by the predetermined method. The results are shown in Fig. 9. In Fig. 9, the vertical axis indicates the assembled area of beads, the assembled area of multilayer portions, or the proportion of multilayer portions, and the horizontal axis indicates the concentration of CEACAM-5 in the liquid sample. Note that, after modification, the final BSA concentration in the antibody-modified bead dispersing solution by washings (three times) was $2.16 \times 10^{-4}$% for the suspension of beads modified by the antibody A, and $1.65 \times 10^{-8}$% for the suspension of beads modified by the antibody B.

Basic composition of dilution solution used

10 mM phosphate buffer (pH 6.3, not containing NaCl and KCl)
BSA concentration of dilution solution used
0.00001%, 0.0001%, 0.001%, or 0.01%

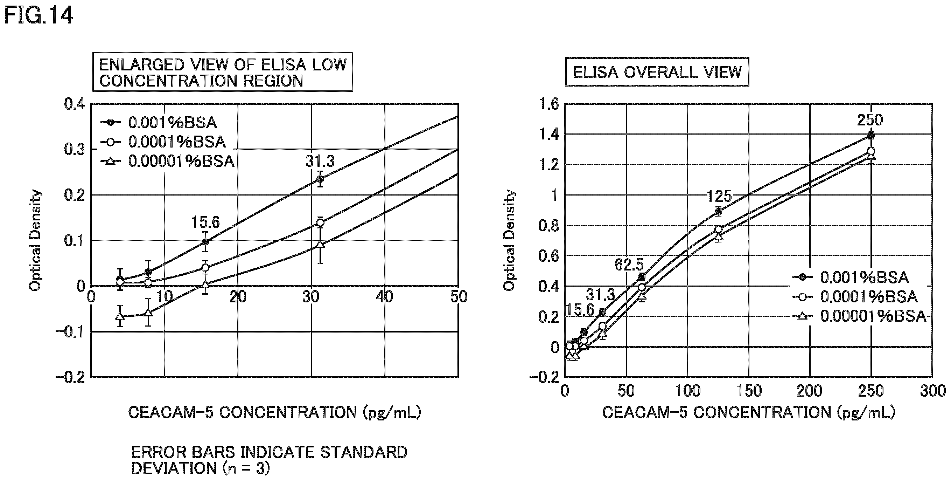

The results in Fig. 9 show that a BSA concentration of 0.0001% can reduce the accumulation of multilayer portions at a CEACAM-5 concentration of 0 pg/mL. This BSA concentration is one order of magnitude lower than the optimal value of concentration as the blocking agent in ELISA (0.001%, see Fig. 14), and the reason for such results is thought to be that BSA was also condensed by optical condensation and efficiently adsorbed on the surface of the antibody-modified beads, thus expressing the blocking effect. Note that the BSA concentration was lower than the optimal concentration of BSA in the ELISA method described later (that is, lower than a concentration at which the non-specific adsorption between the host molecules is inhibited in an environment where a light-induced force does not act on the host molecules).

Fig. 10 is photographs showing the vicinity of the laser spot when the sample is observed at a BSA concentration of 0.0001%. In Fig. 10, the concentration in the upper left corner indicates the concentration of CEACAM-5. The results in Fig. 10 show that the dark-colored region increased depending on the concentration of CEACAM-5.

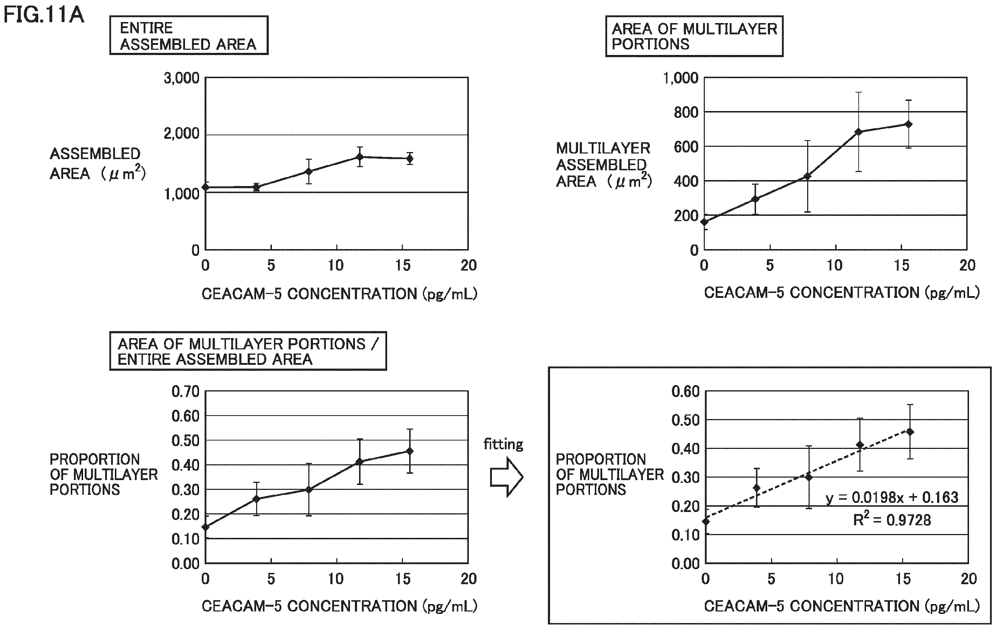

Fig. 11A is graphs showing the correlation between the overall assembled area, the assembled area of multilayer portions, or the proportion of multilayer portions (vertical axis) and the concentration of the glycoprotein in the sample (horizontal axis) when observing the sample at a BSA concentration of 0.0001%. The lower right graph in Fig. 11A is a graph determining the model function between the proportion of multilayer portions (vertical axis) and the concentration of the glycoprotein (CEACAM-5) in the sample (horizontal axis) by the least-squares method based on the measured experimental data. The coefficient of determination ($R^2$) was 0.9728, suggesting that the concentration of the glycoprotein in the sample (in particular, the low concentration range of 0 to 15.6 pg/mL) can be determined with high accuracy based on the proportion of multilayer portions.

<Study 4: Salt concentration-dependence (ionic strength-dependence) of liquid sample>

An experiment was performed by the same method as in Study 1, except that PBS was used as the dilution solution, and the state of assembly of beads was observed in the measurement field of view.

The results of the experiment confirmed that, in the case where PBS was used as the dilution solution, salting out of the beads occurred regardless of the concentration of the analyte (CEACAM-5). On the other hand, in the case where the phosphate buffers were used as in Studies 1 to 3, salting out of the beads was not observed. These results suggest that precipitation of the beads by salting out can be inhibited by reducing the salt concentration (ionic strength) in the measurement sample.

The above results of Study 1, Study 3, and Study 4 show that the dilution solution should be as low as possible in the neutral range, the pH value of the dilution solution should be higher than the isoelectric point of the glycoprotein, the concentration of the blocking agent should be lower than a concentration at which the non-specific adsorption between the host molecules is inhibited in an environment where a light-induced force does not act on the host molecules, and the salt concentration in the dilution solution should be a concentration at which the microparticles modified by the host molecules cannot be precipitated by salting out, which are important to detect a minute amount of the glycoprotein contained in the sample rapidly and with high sensitivity using the optical condensation system.

<Study 5: Study of blocking agent concentration in dispersing solution>

In the experiment of Study 5, the antibody A was diluted with the 10 mM phosphate buffer (pH 6.7) such that the final concentration was 20 μg/mL. The antibody B was diluted with PBS containing 0.57% BSA (pH 7.2 to 7.4) such that the final concentration was 20 μg/mL. By preparing the solution containing the antibody A and the solution containing the antibody B in this manner, both of the solutions contained BSA at a final concentration of 0.56%. Furthermore, after modification, the final BSA concentration in the antibody-modified bead dispersing solution by washings (four times) was reduced by $(1/6)^4$ times, resulting in $0.28 \times (1/6)^5 = 3.6 \times 10^{-5}\%$.

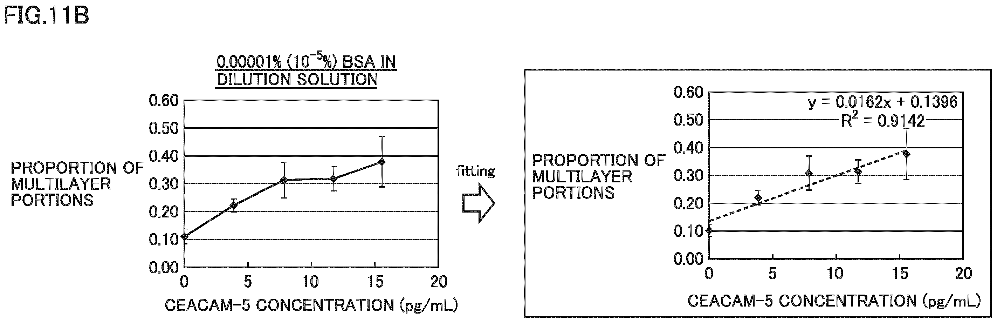

For the subsequent operation, an experiment was performed by the same method as in Study 3 above, except that a phosphate buffer containing 0.00001% BSA was used as the dilution solution so as to have a BSA concentration similar to the BSA concentration in the antibody-modified bead dispersing solution described above, and the suspension of the beads modified by the antibody A and the suspension of the beads modified by the antibody B were each washed four times with a dispersing solution with the following composition, and the assembled area of beads, the assembled area of multilayer portions, and the proportion of multilayer portions in the measurement field of view were determined by the predetermined method. The results are shown in Fig. 11B. In Fig. 11B, the vertical axis indicates the assembled area of beads, the assembled area of multilayer portions, or the proportion of multilayer portions, and the horizontal axis indicates the concentration of CEACAM-5 in the liquid sample.

Composition of dispersing solution used

10 mM phosphate buffer (pH 6.3, not containing NaCl and KCl)
BSA concentration: $1.0 \times 10^{-5}\%$

The results in Fig. 11B show that it was possible to obtain a calibration curve with better linearity when preparing suspensions of beads modified by the antibody A or antibody B using a dispersing solution containing a predetermined concentration of BSA.

<<3. Method for storing sample containing glycoprotein>>

The following experiment was performed to verify whether the method for storing a sample containing a glycoprotein affects quantification of the glycoprotein using an optical condensation system.

At first, the blood was taken from two patients with large bowel carcinoma, and the plasma was collected by a predetermined method. For each plasma obtained, the concentration of CEACAM-5 (marker protein) was determined by ELISA to be 2.4 ng/mL and 13.2 ng/mL, respectively. Hereinafter, plasma with a low concentration of CEACAM-5 will be described as "plasma A", and plasma with a high concentration of CEACAM-5 will be described as "plasma D". The plasma A and plasma D were both stored refrigerated at 4°C for 1 to 4 hours after collection and then stored at -80°C or lower. Note that the plasma A and plasma D correspond to the plasma A and plasma D in the experiment using clinical samples described later.

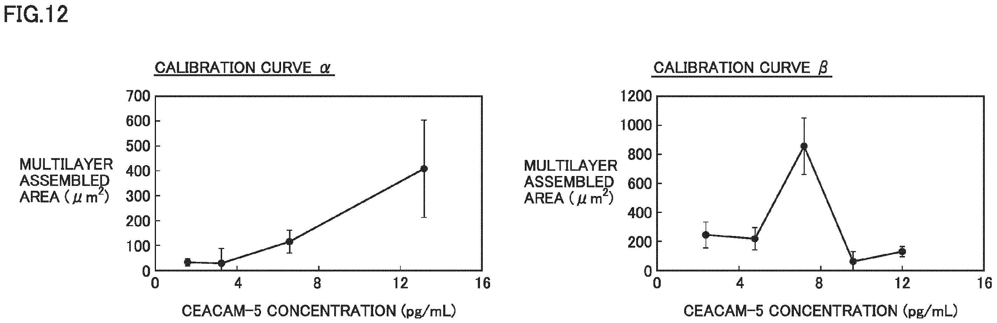

Next, the plasma D was diluted 1000 to 8000 times with a dilution solution having the following composition to prepare four measurement samples with different concentrations of CEACAM-5 (1.6 pg/mL, 3.2 pg/mL, 6.6 pg/mL, and 13.2 pg/mL). An experiment was performed on the four measurement samples prepared by the same method as in Study 1, and the assembled area of beads, the assembled area of multilayer portions, and the proportion of multilayer portions in the measurement field of view were determined by the predetermined method. The results are shown in Fig. 12 (left side, calibration curve $\alpha$). In Fig. 12, the vertical axis indicates the assembled area of multilayer portions, and the horizontal axis indicates the concentration of CEACAM-5.

Composition of dilution solution: 10 mM phosphate buffer (pH 7.2)

Meanwhile, for the plasma A, by diluting it 1000 times with the dilution solution and then adding Recombinant Human CEACAM-5 used in Study 1, five measurement samples with different concentrations of CEACAM-5 were prepared (2.4 pg/mL, 4.8 pg/mL, 7.2 pg/mL, 9.6 pg/mL, and 12.0 pg/mL). An experiment was performed on the five measurement samples prepared by the same method as in Study 1, and the assembled area of beads, the assembled area of multilayer portions, and the proportion of multilayer portions in the measurement field of view were determined by the predetermined method. The results are shown in Fig. 12 (right side, calibration curve $\beta$).

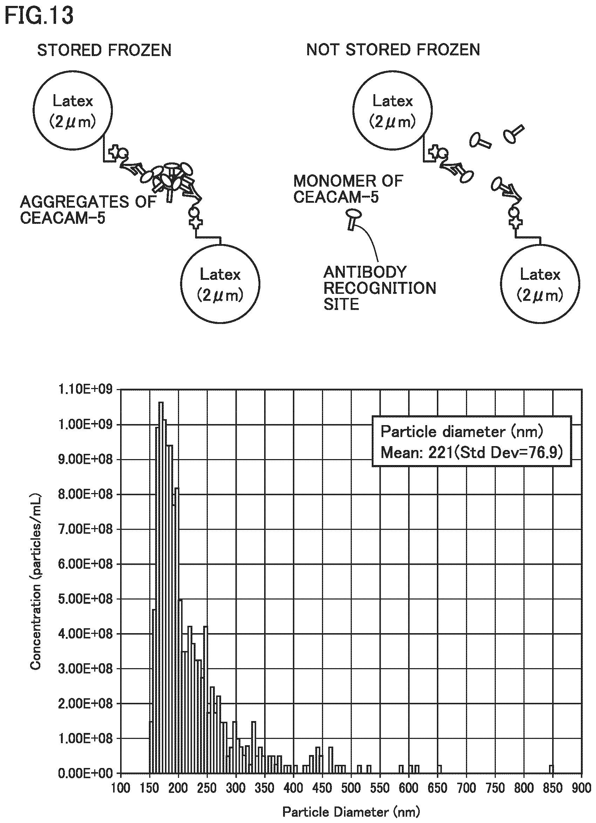

The results in Fig. 12 show that the assembled area of multilayer portions increased depending on the concentration of CEACAM-5 in the experiment using the plasma D. In contrast, no correlation was found between the concentration of CEACAM-5 and the assembled area of multilayer portions in the experiment using the plasma A. In this experiment, the beads were efficiently assembled in the measurement samples prepared from the plasma D, despite that only beads modified by the antibody A were used. Given that CEACAM-5 has a single epitope for the antibody A, it is thought that storing plasma frozen allows CEACAM-5 to form a multimer, contributing to assembly of the beads (figure on the upper left side of Fig. 13). On the other hand, in the measurement samples prepared from the plasma A, Recombinant Human CEACAM-5, which was not stored frozen, was added later. The CEACAM-5 added is considered to be a monomer (monomeric), and it is thought that the beads could not be efficiently assembled (upper right figure of Fig. 13). The above results suggested that the sample containing a glycoprotein is preferably a sample that has been stored frozen for a predetermined period of time after collection.

<<4. Comparative Example: Quantification by ELISA method (low concentration region)>>

It was verified whether the conventional ELISA method could be used to quantify a concentration of 15.6 pg/mL or less. The ELISA kit used was the following.

(ELISA kit)

Human CEACAM-5/CD66e DuoSet ELISA (R&D Systems)
DuoSet Ancillary Reagent Kit 2 (R&D Systems)

Recombinant Human CEACAM-5 used in Study 1 was dissolved in a 10 mM phosphate buffer (pH 6.3). Next, the solution of CEACAM-5 prepared was diluted with a 10 mM phosphate buffer (pH 6.3) in which BSA was dissolved to form samples for measurement (two times dilution series, CEACAM-5 concentration 3.9 pg/mL to 250 pg/mL). At this time, the concentration of BSA contained in the phosphate buffer was set to 0.001%, 0.0001%, or 0.00001%. The absorbance of each sample on a microplate was measured by performing an experiment according to the manual accompanying the ELISA kit. The results are shown in Fig. 14.

In the case where the target protein is quantified by the ELISA method, the optimal absorbance is considered to be 0.1 to 2.0. The results in Fig. 14 show that the absorbance is below 0.1 in the range where the concentration of CEACAM-5 is 15.6 pg/mL or less, indicating that this concentration range is not suited for quantification by the ELISA method.

<<5. Experiment using clinical samples>>

<Comparative study of optical condensation system and conventional method (ELISA method)>

The following experiment was performed to verify whether quantification of a glycoprotein using the optical condensation system is possible even in the case of using plasma samples clinically obtained as the sample.

At first, the blood was taken from four patients with large bowel carcinoma, and the plasma was collected by a predetermined method. For each plasma obtained, the concentration of CEACAM-5 was determined by ELISA to be 2.4 ng/mL, 4.7 ng/mL, 6.3 ng/mL, and 13.2 ng/mL, respectively. Hereinafter, these four types of plasma will be described as "plasma A", "plasma B", "plasma C", and "plasma D" in ascending order in concentration of CEACAM-5. The four types of plasma were all stored refrigerated at 4°C for 1 to 4 hours after collection and then stored at -80°C or lower.

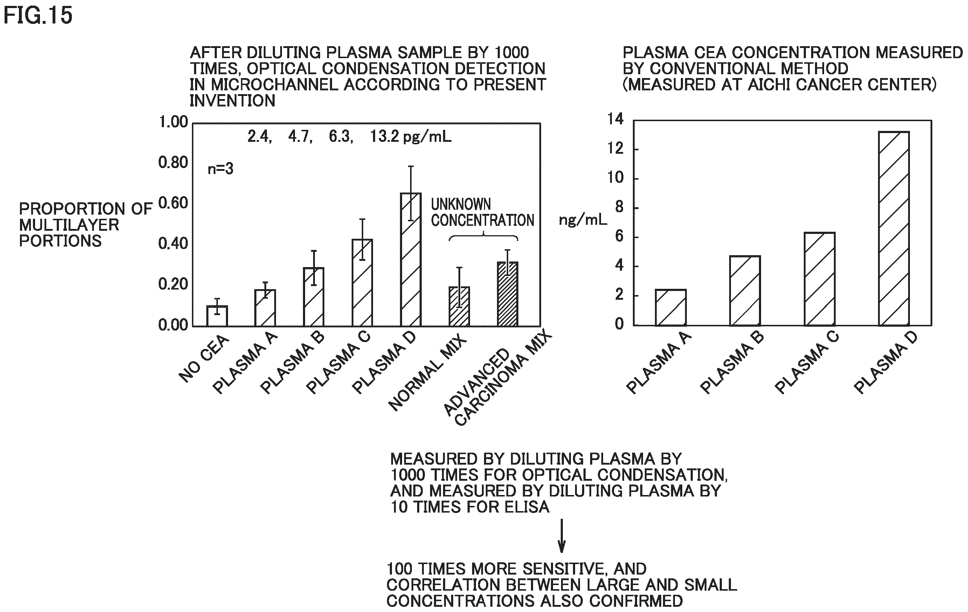

In measurement using the optical condensation system, an experiment was performed by the same method as in Study 1, except that each of the plasma samples obtained was diluted 1000 times using a dilution solution (10 mM phosphate buffer (pH 7.2)), and the proportion of multilayer portions in the measurement field of view was determined by the predetermined method. The results are shown in Fig. 15 (left side).

As a comparative experiment, the same measurement was performed by the conventional ELISA method. In the ELISA method, an experiment was performed by the same method as in "4. Comparative Example: Quantification by ELISA method (low concentration region)", except that each of the plasma samples obtained was diluted 10 times using a dilution solution (10 mM phosphate buffer (pH 7.2)) for use, and the concentration of CEACAM-5 in the plasma samples was determined. The results are shown in Fig. 15 (right side). These experimental results show that measurement using the optical condensation system is 100 times more sensitive than the conventional method, and can also confirm the correlation between large and small CEACAM-5 concentrations among multiple plasma samples.

<Measurement of clinical samples using kit according to present embodiment>

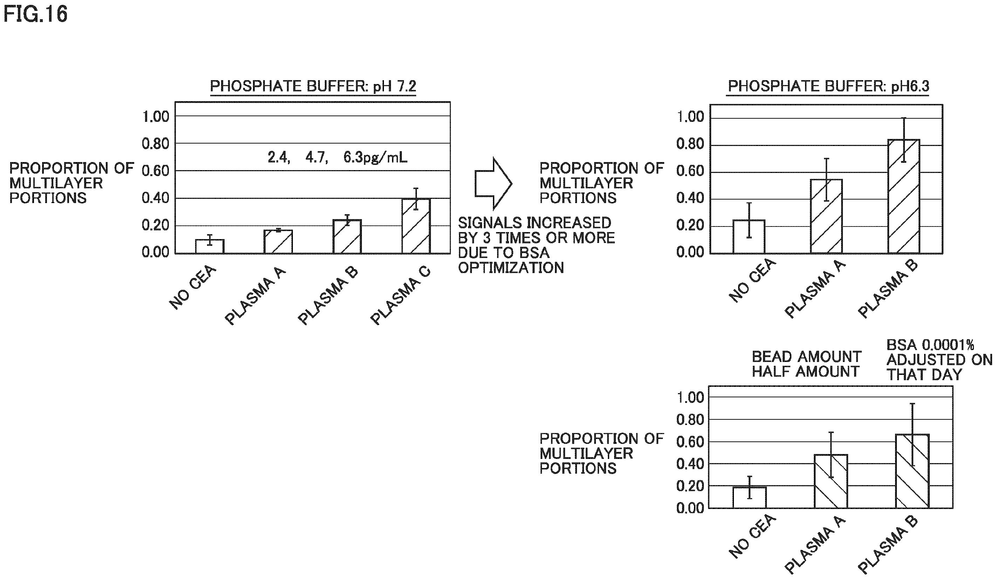

For the plasma A and plasma B described above, measurement by the optical condensation system was performed after dilution with the dilution solution according to the present embodiment. That is, an experiment was performed by the same method as in Study 3, except that each of the plasma A and plasma B was diluted 1000 times with a dilution solution (10 mM phosphate buffer, pH 6.3, 0.0001% BSA) and used as the measurement sample, and the proportion of multilayer portions in the measurement field of view was determined by the predetermined method. The results are shown in Fig. 16 (upper right graph). Note that the lower right graph in Fig. 16 shows the results when the same experiment was performed with half the amount of each of the suspension of the beads modified by the antibody A and the suspension of the beads modified by the antibody B as used in Study 3. Also, the graph on the left side in Fig. 16 is an excerpt of the graph on the left side in Fig. 15.

The experimental results in Fig. 16 show that, by lowering the pH value of the dilution solution used and optimizing the BSA added, the detection signals (proportion of multilayer portions) increased by three times or more, leading to improvement in detection sensitivity.

<<6. Effect of surface potential of beads on non-specific adsorption>>

The following experiment was performed in order to study how the surface potential of beads affects the non-specific adsorption between antibodies modifying the beads.

<Measurement of zeta potential of beads>

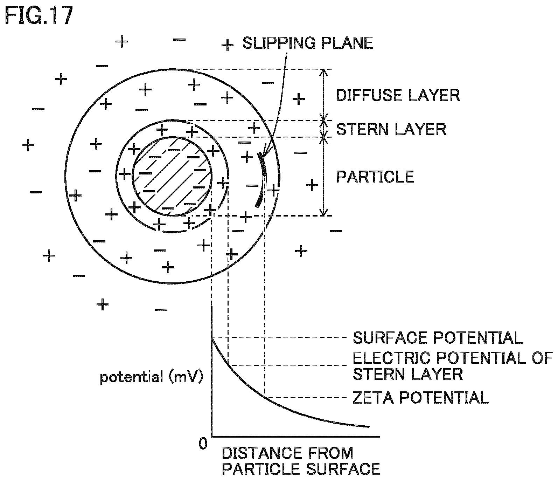

At first, as a preliminary experiment, the following two types of beads were prepared to measure the zeta potential of the beads (as shown in Fig. 17, the zeta potential serves as an indicator representing the thickness of the electrical double layer composed of the Stern layer and the diffuse layer, as well as the intensity of the surface potential). Note that non-fluorescent beads are the beads used in the sections of " 1. Fabrication of beads modified by antibodies" to "3. Method for storing sample containing glycoprotein" and "5. Experiment using clinical samples" described above.

(Beads (microparticles))

Non-fluorescent beads: Streptavidin Coated Microsphere 2 $\mu$m Plain (manufactured by Polysciences, Inc., Cat. No. 24160, material: polystyrene)
Fluorescent beads: Streptavidin Fluoresbrite YG Coated Microsphere 2 $\mu$m (manufactured by Polysciences, Inc., Cat. No. 24159, material: polystyrene)

The measurement device used was the zeta potential, particle size, and molecular weight measurement system ELSZneo (product name, manufactured by Otsuka Electronics Co., Ltd.). The measurement conditions are as follows. The results are shown in Table 1.

(Measurement conditions for zeta potential)

Cell type: zeta potential micro volume disposable cell
Solvent: phosphate buffer (10 mM, pH 6.7)
Temperature: 25°C
Applied voltage: -24.54 (V)

[Table 1]

| | Zeta potential (mV) | | | |
|---|---|---|---|---|
| | First time | Second time | Third time | Average (standard deviation) |
| Non-fluorescent beads | -36.16 | -36.93 | -36.85 | -36.65 (S.D.=0.43) |
| Fluorescent beads | -52.47 | -50.62 | -51.70 | -51.6 (S.D.=0.93) |

The results in Table 1 show that the non-fluorescent beads have a negative zeta potential, while the fluorescent beads have a strong negative zeta potential, resulting in strong electrostatic repulsion between the beads.

<Study on antibody modification amount when using fluorescent beads>

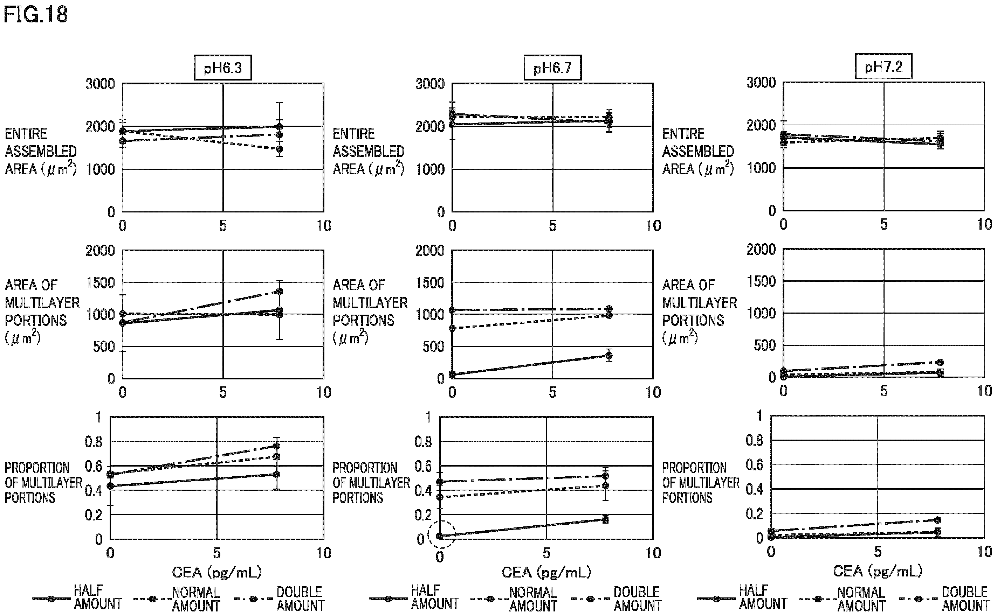

Since the fluorescent beads have a surface potential (zeta potential) different from that of the non-fluorescent beads, it was studied how the assembled area of beads, the assembled area of multilayer portions, and other properties are affected when changing the antibody modification amount (Fig. 18). Antibody modification on the fluorescent beads was performed in accordance with the section of " 1. Fabrication of beads modified by antibodies" described above. At this time, the solution containing the antibody A and the solution containing the antibody B were prepared by dilution with a 10 mM phosphate buffer (pH 6.7, containing 0.00001% BSA) such that the final concentration of each antibody was 10 $\mu$g/mL (half amount), 20 $\mu$g/mL (normal amount), and 40 $\mu$g/mL (double amount). By this method, suspensions of the beads with an antibody modification amount of "half amount", "normal amount", or "double amount" were prepared.

(Preparation of liquid sample)

Recombinant Human CEACAM-5 (glycoprotein) (concentration 100 ng/mL) packaged together in Human CEACAM-5/CD66e DuoSet ELISA (manufactured by R&D Systems, Cat. No. DY4128, kit for ELISA) was diluted with the dilution solution described later to a final concentration of 7.8 pg/mL, thereby preparing a standard sample. For the dilution solution, three different phosphate buffers with different pH values were used (pH: 7.2, 6.7, and 6.3). The phosphate buffers were prepared by diluting commercially available phosphate buffers (phosphate buffers described above manufactured by FUJIFILM Wako Pure Chemical Corporation) 10 times with DDW. Also, as standard samples with a CEACAM-5 concentration of 0 pg/mL, the three dilution solutions described above were used as they were. That is, two standard samples with different CEACAM-5 concentrations were prepared for each phosphate buffer, which thus means that six standard samples with different pH values and CEACAM-5 concentrations were prepared.

20 $\mu$L of each of the six standard samples was transferred to microtubes, to which the suspension of fluorescent beads modified by the antibody A (10 $\mu$L) and the suspension of fluorescent beads modified by the antibody B (10 $\mu$L) described above were added and mixed well. By the above procedure, eighteen measurement samples (pH 6.3 to 7.2, CEACAM-5 concentrations of 0 pg/mL or 7.8 pg/mL, antibody modification amount "half amount", "normal amount", or "double amount") were prepared.

(Detection by optical condensation system)

The prepared measurement samples were injected into the microchannel of the microchannel chip in the optical condensation system, and the measurement samples were irradiated with light under the following conditions. Thereafter, the assembled area of beads, the assembled area of multilayer portions, and the proportion of multilayer portions (hereinafter, "multilayer proportion") in the measurement field of view were determined by the methods described above. The results are shown in Fig. 18. In Fig. 18, the vertical axis indicates the assembled area of fluorescent beads, the assembled area of multilayer portions, or the proportion of multilayer portions, and the horizontal axis indicates the concentration of CEACAM-5 in the measurement sample.

Conditions for irradiation with light

Microchannel: 100 $\mu$m $\times$ 100 $\mu$m
Syringe: inner diameter 2.3 mm / volume 0.25 mL
Flow rate: 250 $\mu$L syringe setting, 0.1 $\mu$L/min
Laser intensity: measured at 30 sec after 265 mW irradiation for 5 min
Position of beam waist of x40 lens: 45 $\mu$m below the bottom of channel

The results in Fig. 18 show that, for the fluorescent beads, the proportion of multilayer portions at a CEACAM-5 concentration of 0 pg/mL is reduced to the lowest at pH 6.7 and when the antibody modification amount is half amount. These results indicate that non-specific adsorption between the fluorescent beads can be sufficiently inhibited.

<Study on calibration curve when using fluorescent beads>

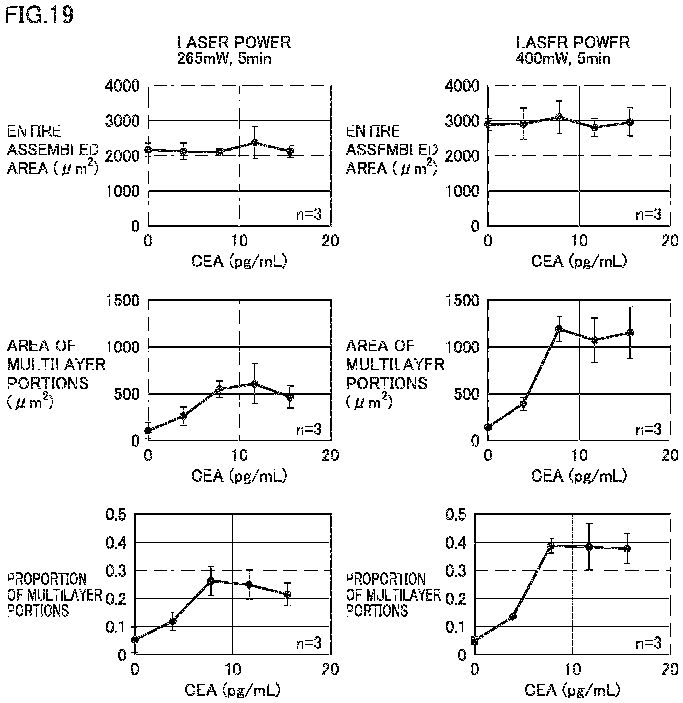

Measurement samples were prepared by the same method as in the column of (Preparation of liquid sample) in <Study on antibody modification amount when using fluorescent beads> described above, except that a phosphate buffer (10 mM) with a pH of 6.7 was used as the dilution solution, suspensions with an antibody modification amount of "half amount" were used as the suspensions of fluorescent beads for both antibody A and antibody B, and standard samples (5 types) with a CEACAM-5 concentration of 0 to 15.6 pg/mL were used. The prepared measurement samples were injected into the microchannel of the microchannel chip in the optical condensation system, and the measurement samples were irradiated with light under the following conditions. Thereafter, the assembled area of beads, the assembled area of multilayer portions, and the proportion of multilayer portions in the measurement field of view were determined by the method described above. The results are shown in Fig. 19. In Fig. 19, the vertical axis indicates the assembled area of fluorescent beads, the assembled area of multilayer portions, or the proportion of multilayer portions, and the horizontal axis indicates the concentration of CEACAM-5 in the measurement sample.

Conditions for irradiation with light

Microchannel: 100 μm × 100 μm
Syringe: inner diameter 1.03 mm / volume 50 μL
Flow rate: 50 μL syringe setting, 0.1 μL/min
Laser intensity: measured at 30 sec after 265 mW or 400 mW irradiation for 5 min
Position of beam waist of x40 lens: 45 μm below the bottom of channel

In Fig. 11A and Fig. 11B (experimental results using non-fluorescent beads), the proportion of multilayer portions was about 0.4 when the concentration of CEACAM-5 was 15.6 pg/mL, while in Fig. 19, the proportion of multilayer portions reached 0.4 when the concentration of CEACAM-5 was 7.8 pg/mL. These results show that the use of fluorescent beads allows for detection at a lower concentration. Also, it is shown that, by increasing the laser intensity from 265 mW to 400 mW, the light-induced force is enhanced and efficiently pushes the beads against the bottom of the channel, thus increasing the entire assembled area, the area of multilayer portions, and the proportion of multilayer portions, and obtaining a calibration curve with a large slope in the low concentration region.

<Experiment using human CA19-9 as glycoprotein>

The following experiment was performed to study whether quantification of a glycoprotein using the optical condensation system is also possible even in the case of using human CA19-9 as the glycoprotein. The experiment was performed under the same conditions as in <Study on calibration curve when using fluorescent beads> described above, except that biotinylated anti-human CA19-9 antibody was used as the antibody A, the fluorescent beads modified by the antibody A used had a modification amount of "double amount", human CA19-9 was used as the glycoprotein, and the laser intensity was set to 400 mW. Note that the biotinylated anti-human CA19-9 antibody and human CA19-9 used were those packaged together in the CA19-9 ELISA Kit, Human, RayBio (1 × 96 well strip plate) (manufactured by RayBiothch, Inc., Cat. No. ELH-CA19-91). The results are shown in Table 2.

[Table 2]

| CA19-9 [U/mL] | 0.000 | 0.125 | 0.250 | 0.500 |
|---|---|---|---|---|
| Entire assembled area* | 2211.28 | 2397.22 | 2195.15 | 2177.64 |
| Area of multilayer portions* | 921.38 | 1171.01 | 1213.03 | 1219.87 |
| Proportion of multilayer portions* | 0.42 | 0.49 | 0.56 | 0.56 |
| * The average value at n = 3 is shown. | | | | |

The results in Table 2 show that there is a positive correlation between the concentration of the glycoprotein and the area of multilayer portions and the proportion of multilayer portions, even in the case of using human CA19-9 as the glycoprotein. That is, it is shown that quantification of a glycoprotein using the optical condensation system is also possible even in the case of using human CA19-9 as the glycoprotein.

<Experiment using clinical samples>

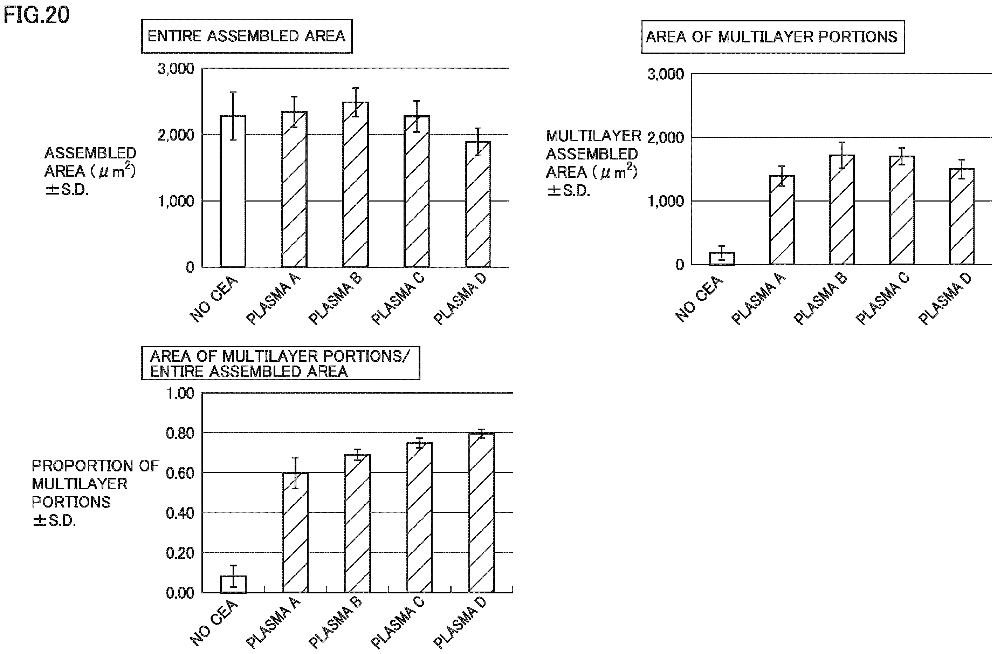

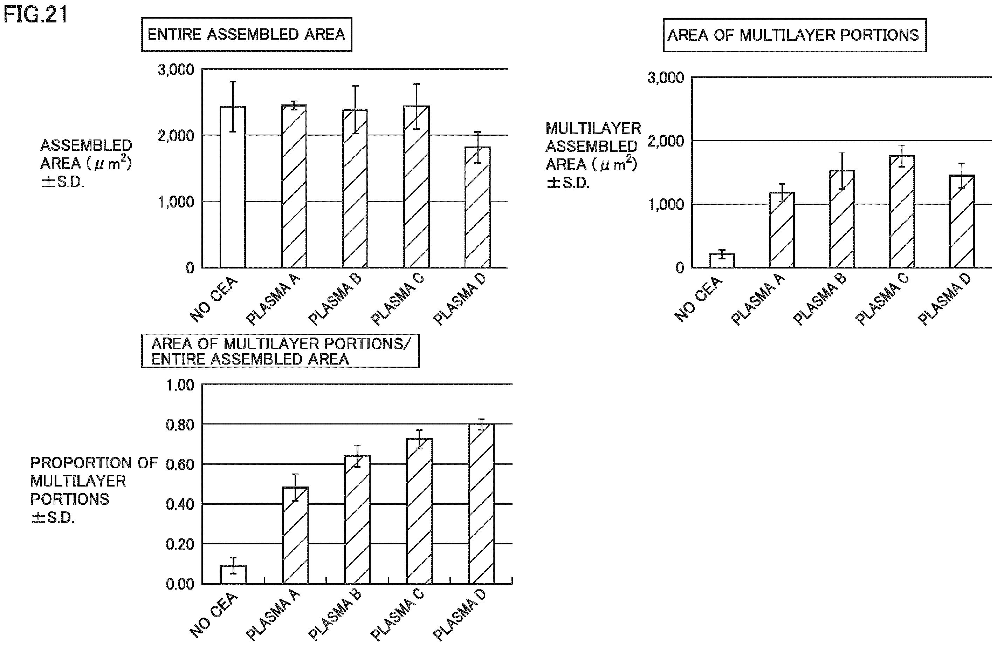

The following experiment was performed to verify whether it is possible to quantify a glycoprotein in plasma samples clinically obtained even in an optical condensation system using fluorescent beads (Figs. 20 and 21). The plasma samples used were "plasma A", "plasma B", "plasma C", and "plasma D", which were used in the section of "5. Experiment using clinical samples" described above.

In measurement using the optical condensation system, an experiment was performed by the same method as in the section of "Study on calibration curve when using fluorescent beads" described above, except that each of the plasma samples obtained was diluted 1000 times using a dilution solution (10 mM phosphate buffer, pH 6.7, containing 0.00001% BSA), the laser intensity was set to 400 mW, and the laser irradiation time was set to 5 min or 4 min, and the assembled area of fluorescent beads, the assembled area of multilayer portions, and the proportion of multilayer portions in the measurement field of view were determined by the predetermined method. The results are shown in Figs. 20 and 21. In Figs. 20 and 21, the vertical axis indicates the assembled area of fluorescent beads, the assembled area of multilayer portions, or the proportion of multilayer portions, and the horizontal axis indicates the label of the plasma sample.

The results in Figs. 20 and 21 show a correlation between the CEA concentration in the plasma sample and the proportion of multilayer portions, even in the case of using fluorescent beads. In particular, the correlation was clearer in the case where the laser irradiation time was set to 4 min.

<<7. Effect of improving specificity by salt concentration (ionic strength) in liquid sample>>

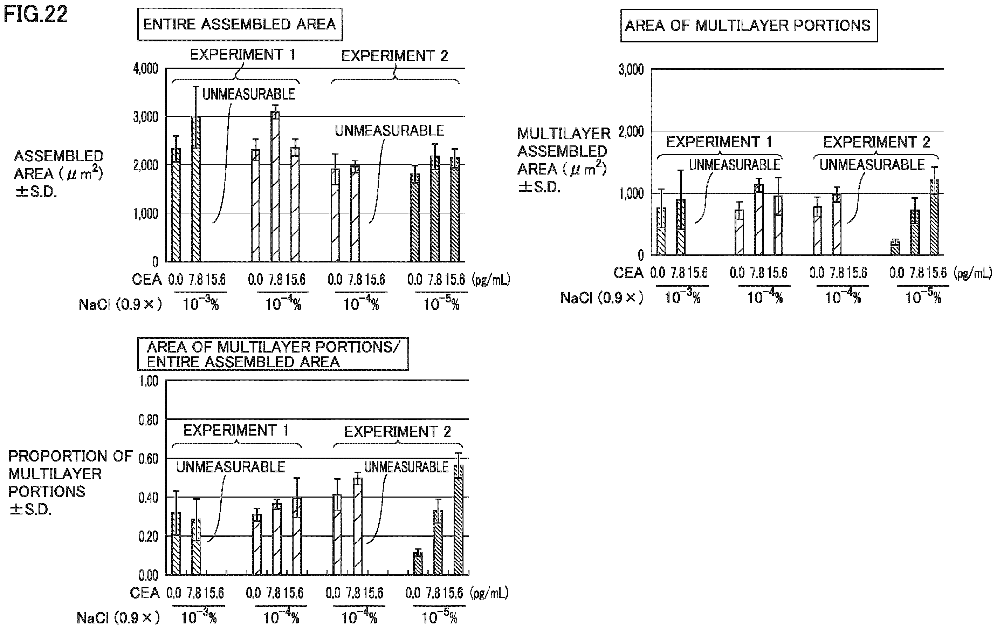

In Study 4, it is confirmed that the beads are salted out when the salt concentration of the liquid sample is too high. In this experiment, it was studied in further detail whether there is a salt concentration optimal for measurement using the optical condensation system at which salting out of the beads does not occur (Fig. 22).

<Experiment 1>

Measurement samples were prepared by the same method as in the column of (Preparation of liquid sample) in <Study on antibody modification amount when using fluorescent beads> described above, except that the following two phosphate buffers with different NaCl concentrations were used as the dilution solution, suspensions with an antibody modification amount of "half amount" for the antibody A and with an antibody modification amount of "normal amount" for the antibody B were used as the suspensions of fluorescent beads, and standard samples (3 types, 10 $\mu$L) with a CEACAM-5 concentration of 0 to 15.6 pg/mL were used. The prepared measurement samples were injected into the microchannel of the microchannel chip in the optical condensation system, and the measurement samples were irradiated with light under the following conditions. Thereafter, the assembled area of beads, the assembled area of multilayer portions, and the proportion of multilayer portions in the measurement field of view were determined by the method described above. The results are shown in Fig. 22. In Fig. 22, the vertical axis indicates the assembled area of fluorescent beads, the assembled area of multilayer portions, or the proportion of multilayer portions, and the horizontal axis indicates the concentration of CEACAM-5 and the sodium chloride concentration in the measurement sample.

Basic composition of dilution solution used

10 mM phosphate buffer (pH 6.7, containing 0.00001% BSA)
NaCl concentration of dilution solution used

$$0.9 \times 10^{-3}\% \text{ (w/v) or } 0.9 \times 10^{-4}\% \text{ (w/v)}$$

Conditions for irradiation with light

Microchannel: 100 $\mu$m $\times$ 100 $\mu$m
Syringe: inner diameter 1.03 mm / volume 50 $\mu$L
Flow rate: 50 $\mu$L syringe setting, 0.1 $\mu$L/min
Laser intensity: 400 mW irradiation for 5 min
Position of beam waist of x40 lens: 45 $\mu$m below the bottom of channel

<Experiment 2>

An experiment was performed by the same method as in Experiment 1 above, except that the NaCl concentration in the dilution solution was set to 0.9 $\times$ 10$^{-4}$% (w/v) or 0.9 $\times$ 10$^{-5}$% (w/v), a different lot was used for the suspension of fluorescent beads modified by the antibody B than in Experiment 1 above, and the "flow rate" in the light irradiation conditions was changed to 0.1 $\mu$L/min, 0.15 $\mu$L/min, or 0.2 $\mu$L/min, depending on the situation, and the assembled area of fluorescent beads, the assembled area of multilayer portions, and the proportion of multilayer portions in the measurement field of view were determined by the predetermined method. The results are shown in Fig. 22.

The results in Fig. 22 show that, in the case where the NaCl concentration in the dilution solution was 0.9 $\times$ 10$^{-5}$% (w/v), there was less non-specific adsorption between the fluorescent beads at a CEACAM-5 concentration of 0 pg/mL, and the area of multilayer portions and the proportion of multilayer portions increased in correlation with the CEACAM-5 concentration (results of Experiment 2 in Fig. 22). More specifically, the proportion of multilayer portions was saturated at 0.4 at CEA concentrations of 7.8 pg/mL or more in Fig. 19, whereas the proportion of multilayer portions increased to 0.6 even at 15.6 pg/mL in the NaCl concentration of 0.9 $\times$ 10$^{-5}$% (w/v) in Fig. 22. This indicates that addition of NaCl to the extent that salting out does not occur reduces the absolute value of zeta potential (the thickness of the electrical double layer in Fig. 17 is also reduced), which makes it easier for CEA and the antibody to approach each other and allows the antigen-antibody reaction to occur more efficiently.

<<8. Blocking in microchannel>>

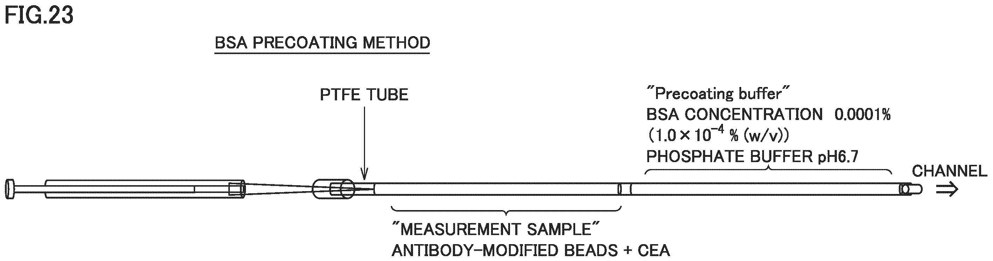

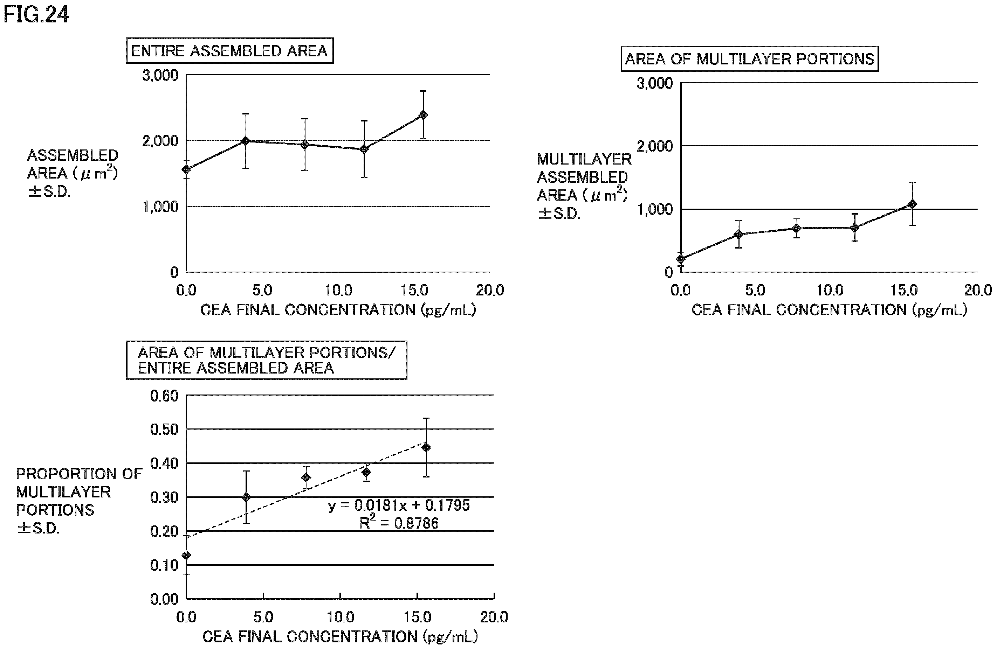

It was studied whether blocking (pre-coating) of the inside of the microchannel with BSA or other materials improves measurement accuracy (Fig. 23 and Fig. 24). The specific procedure is as follows.

At first, the inside of a polytetrafluoroethylene tube (PTFE tube) into which the measurement sample was to be suctioned was filled with a precoating buffer (10 mM phosphate buffer, pH 6.7, 0.0001% BSA,) which was then discharged, thereby blocking the inside of the PTFE tube with BSA.

Measurement samples were prepared by the same method as in Experiment 2 in the column of (7. Effect of improving specificity by salt concentration (ionic strength) in liquid sample) described above, except that the NaCl concentration in the dilution solution was set to $0.9 \times 10^{-5}$% (w/v) and standard samples (5 types, 20 $\mu$L) with a CEACAM-5 concentration of 0 to 15.6 pg/mL were used. Next, the measurement sample (20 $\mu$L) was suctioned into the PTFE tube, followed successively by suction of the precoating buffer (20 $\mu$L) (see Fig. 23). The PTFE tube was set in a syringe pump and injected into the microchannel, and the measurement sample was irradiated with light under the following conditions. Thereafter, the assembled area of beads, the assembled area of multilayer portions, and the proportion of multilayer portions in the measurement field of view were determined by the method described above. The results are shown in Fig. 24. In Fig. 24, the vertical axis indicates the assembled area of fluorescent beads, the assembled area of multilayer portions, or the proportion of multilayer portions, and the horizontal axis indicates the concentration of CEACAM-5 in the measurement sample.

Conditions for irradiation with light

Microchannel: 100 $\mu$m $\times$ 100 $\mu$m
Syringe: inner diameter 1.03 mm / volume 50 $\mu$L
Flow rate: 50 $\mu$L syringe setting, 0.15 $\mu$L/min (note that, when the precoating buffer was injected, the flow rate was 4 $\mu$L/min.)
Laser intensity: 400 mW irradiation for 5 min
Position of beam waist of x40 lens: 45 $\mu$m below the bottom of channel

The results in Fig. 24 show that blocking the inside of the microchannel in advance prior to measurement allows for a more stable flow rate when injecting the measurement sample, and allows for creation of a calibration curve with a smaller error margin.

<<9. Stabilization of multilayer structure by mixing beads with different sizes>>

<Preparation of suspensions of fluorescent beads modified by antibodies>

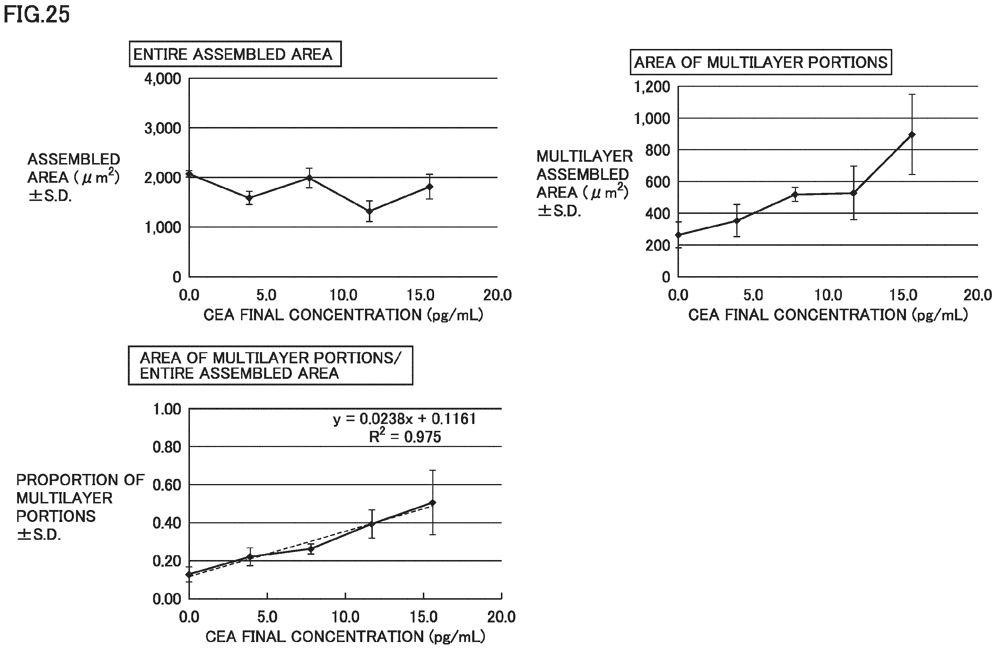

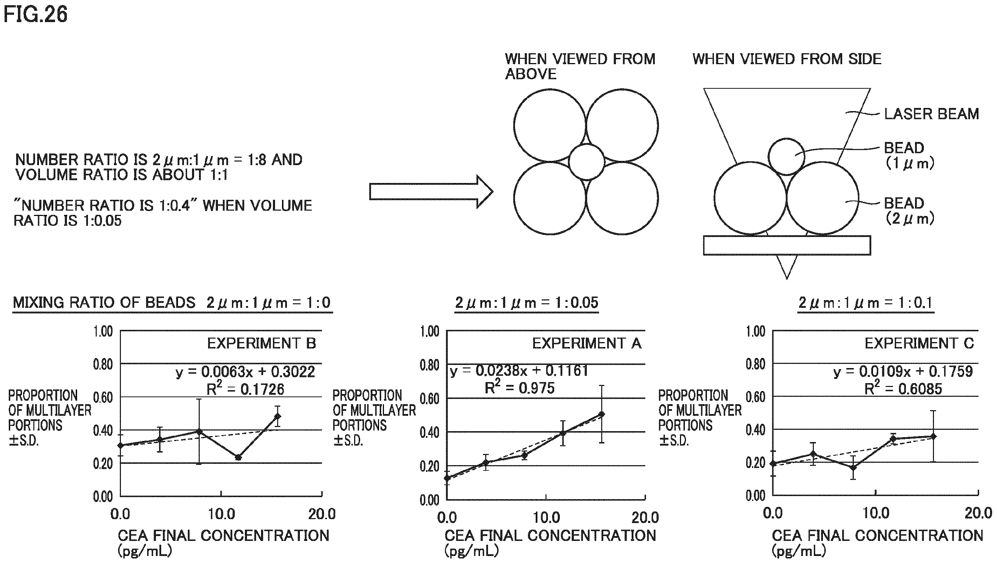

It was studied by the following experiment whether the multilayer structure is stabilized by mixing beads with different sizes (Fig. 25 and Fig. 26). At first, as fluorescent beads modified by antibodies, the following four suspensions of beads, A1, A2, B1, and B2, were prepared. Antibody modification to the fluorescent beads was performed in accordance with the section of "Study on antibody modification amount when using fluorescent beads" described above.

(Suspensions of fluorescent beads modified by antibodies)

(A1) Fluorescent beads modified by antibody A (size: 1 $\mu$m, antibody modification amount: half amount, density in suspension: $4.2 \times 10^9$/mL)
(A2) Fluorescent beads modified by antibody A (size: 2 $\mu$m, antibody modification amount: half amount, density in suspension: $5.3 \times 10^8$/mL)
(B1) Fluorescent beads modified by antibody B (size: 1 $\mu$m, antibody modification amount: normal amount, density in suspension: $4.2 \times 10^9$/mL)
(B2) Fluorescent beads modified by antibody B (size: 2 $\mu$m, antibody modification amount: quadruple amount, density in suspension: $5.3 \times 10^8$/mL)

<Experiment A>

Using a 10 mM phosphate buffer (pH 6.7, containing 0.00001% BSA) as the dilution solution, standard samples (5 types) with a CEACAM-5 concentration of 0 to 15.6 pg/mL were prepared. 20 μL of each of the five standard samples were transferred to microtubes, to which the suspension A1 (5 μL of one diluted by 20 times), suspension A2 (5 μL), suspension B1 (5 μL of one diluted by 20 times), and suspension B2 (5 μL) described above were added and mixed well. By the above procedure, measurement samples were prepared. In the measurement samples prepared in Experiment A, the ratio between the number of 2 μm beads and 1 μm beads was calculated to be about 1:0.4, and the volume ratio was calculated to be about 1:0.05.

The prepared measurement samples were injected into the microchannel of the microchannel chip in the optical condensation system, and the measurement samples were irradiated with light under the following conditions. Thereafter, the assembled area of beads, the assembled area of multilayer portions, and the proportion of multilayer portions in the measurement field of view were determined by the method described above. The results are shown in Fig. 25. In Fig. 25, the vertical axis indicates the assembled area of fluorescent beads, the assembled area of multilayer portions, or the proportion of multilayer portions, and the horizontal axis indicates the concentration of CEACAM-5 in the measurement sample.

Conditions for irradiation with light

Microchannel: 100 μm × 100 μm
Syringe: inner diameter 1.03 mm / volume 50 μL
Flow rate: 50 μL syringe setting, 0.1 μL/min
Laser intensity: 400 mW irradiation for 5 min
Position of beam waist of x40 lens: 45 μm below the bottom of channel

<Experiment B>

Using a 10 mM phosphate buffer (pH 6.7, containing 0.00001% BSA) as the dilution solution, standard samples (5 types) with a CEACAM-5 concentration of 0 to 15.6 pg/mL were prepared. 20 μL of each of the five standard samples were transferred to microtubes, to which the suspension A2 (10 μL) and suspension B2 (10 μL) described above were added and mixed well. In Experiment B, the suspension A1 and suspension B1 were not used. That is, in the measurement samples prepared in Experiment B, the ratio between the number of 2 μm beads and 1 μm beads was 1:0 (the volume ratio was also 1:0). By the above procedure, measurement samples were prepared. The prepared measurement samples were injected into the microchannel of the microchannel chip in the optical condensation system, and the measurement samples were irradiated with light under the same conditions as in Experiment A. Thereafter, the assembled area of beads, the assembled area of multilayer portions, and the proportion of multilayer portions in the measurement field of view were determined.

<Experiment C>

Using a 10 mM phosphate buffer (pH 6.7, containing 0.00001% BSA) as the dilution solution, standard samples (5 types) with a CEACAM-5 concentration of 0 to 15.6 pg/mL were prepared. 20 μL of each of the five standard samples were transferred to microtubes, to which the suspension A1 (5 μL of one diluted by 10 times), suspension A2 (5 μL), suspension B1 (5 μL of one diluted by 10 times), and suspension B2 (5 μL) described above were added and mixed well. By the above procedure, measurement samples were prepared. In the measurement samples prepared in Experiment C, the ratio between the number of 2 μm beads and 1 μm beads was calculated to be about 1:0.8, and the volume ratio was calculated to be about 1:0.1. The prepared measurement samples were injected into the microchannel of the microchannel chip in the optical condensation system, and the measurement samples were irradiated with light under the same conditions as in Experiment A. Thereafter, the assembled area of beads, the assembled area of multilayer portions, and the proportion of multilayer portions in the measurement field of view were determined.

The comparative results of the proportion of multilayer portions determined in Experiments A, B, and C are shown in Fig. 26. In Fig. 26, the "MIXING RATIO OF BEADS" is expressed as a volume ratio. The results in Fig. 26 show that, when the ratio between the number of 2 μm beads and 1 μm beads is about 1:0.4 (1:0.05 in volume ratio) (Experiment A), there is less non-specific adsorption between the beads and a stable multilayer structure is formed.

The embodiments and Examples disclosed here should be considered merely illustrative and not restrictive in all respects. The scope of the present invention is presented by the claims rather than the embodiments and Examples given above, and it is intended that all modifications within the meaning and scope equivalent to the claims be included.

REFERENCE SIGNS LIST

B1, B2, B3 Bead; X, Y Analyte; B0 Bead body; B11 First antibody; B21 Second antibody; B12, B22 Avidin; B13, B23 Biotin; B31 Antibody; 90 Microchannel chip; 91 Substrate; 92 Microchannel; 921 Inlet; 922 Outlet.

**Claims**

1. A kit for detecting a glycoprotein contained in a sample using an optical condensation system, the kit comprising:

   microparticles modified by host molecules; and
   a dilution solution for diluting the sample,
   wherein each of the host molecules binds specifically to the glycoprotein,
   the dilution solution comprises a blocking agent and a buffering agent,
   the pH value of the dilution solution is higher than the isoelectric point of the glycoprotein,
   the concentration of the blocking agent is lower than a concentration at which the non-specific adsorption between the host molecules is inhibited in an environment where a light-induced force does not act on the host molecules, and
   the salt concentration in the dilution solution is a concentration at which the microparticles modified by the host molecules cannot be precipitated by salting out.

2. The kit according to claim 1, wherein the dilution solution is neutral.

3. The kit according to claim 1 or 2, wherein the salt concentration in the dilution solution is a concentration at which the microparticles modified by the host molecules cannot be precipitated by salting out, and at which the thickness of an electrical double layer in the microparticles is reduced.

4. The kit according to claim 1 or 2, wherein the microparticles further comprise an additive that increases an electrostatic repulsive force.

5. The kit according to claim 1 or 2, wherein the microparticles comprise two or more types of microparticles with different sizes.

6. The kit according to claim 1 or 2, wherein the host molecules comprise at least one selected from the group consisting of an antibody, a Fab fragment, a $F(ab')_2$ fragment, a Fv fragment, and a scFv.

7. The kit according to claim 1 or 2, wherein the blocking agent comprises at least one selected from the group consisting of albumin, gelatin, casein, and goat serum.

8. The kit according to claim 1 or 2, wherein the concentration of the blocking agent is 0.000001% by mass or more and less than 0.001% by mass with respect to the dilution solution.

9. The kit according to claim 1 or 2, wherein the buffering agent comprises at least one selected from the group consisting of a phosphate compound, trishydroxymethylaminomethane, HEPES, and MES.

10. The kit according to claim 1 or 2, wherein the kit further comprises a dispersing solution for dispersing the microparticles modified by the host molecules,

    the dispersing solution comprises the blocking agent and the buffering agent, and
    the concentration of the blocking agent is 0.00000001% by mass or more and 0.001% by mass or less with respect to the dispersing solution.

11. The kit according to claim 1 or 2, wherein the glycoprotein is a cancer marker protein.

12. The kit according to claim 11, wherein the cancer marker protein comprises at least one selected from the group consisting of a carcinoembryonic antigen and a carbohydrate antigen.

13. The kit according to claim 1 or 2, wherein the sample is a sample that has been frozen after collection or has been refrigerated for a predetermined period of time and then stored frozen, and
the glycoprotein contained in the sample aggregates to form a multimer or a nanoparticle.

14. The kit according to claim 1 or 2, further comprising a microchannel chip.

15. A kit for detecting a glycoprotein contained in a sample using an optical condensation system, the kit comprising:

microparticles modified by host molecules;
a dilution solution for diluting the sample; and
a dispersing solution for dispersing the microparticles modified by the host molecules,
wherein each of the host molecules binds specifically to the glycoprotein,
the dilution solution comprises a buffering agent,
the dispersing solution comprises a blocking agent and the buffering agent,
the pH value of the dilution solution is higher than the isoelectric point of the glycoprotein,
the concentration of the blocking agent is lower than a concentration at which the non-specific adsorption between the host molecules is inhibited in an environment where a light-induced force does not act on the host molecules, and
the salt concentration in the dilution solution is a concentration at which the microparticles modified by the host molecules cannot be precipitated by salting out.

## FIG.1

## FIG.2

FIG.3

<DOWNWARD IRRADIATION>

FIG.4

ANTIGEN-ANTIBODY REACTION

EP 4 517 323 A1

FIG.5

ANTIGEN–ANTIBODY REACTION

FIG.6

DISSIPATIVE FORCE

SP

L1

B1 (B2)

92

LIQUID FLOW

FIG.7

EP 4 517 323 A1

FIG.8

265 mW, 65 μm DOWNWARD, PHOSPHATE BUFFER A (pH 6.7)

ASSEMBLED AREA ($\mu\,m^2$)

CEACAM-5 CONCENTRATION (pg/mL)

n=3

MULTILAYER ASSEMBLED AREA ($\mu\,m^2$)

y = 114.2x + 377.02
$R^2$ = 0.9606

n=3

CEACAM-5 CONCENTRATION (pg/mL)

PROPORTION OF MULTILAYER PORTIONS

n=3

y = 0.0339x + 0.1545
$R^2$ = 0.9323

CEACAM-5 CONCENTRATION (pg/mL)

265 mW, 65 μm DOWNWARD, PHOSPHATE BUFFER B (pH 6.7 to 6.8)

ASSEMBLED AREA ($\mu\,m^2$)

CEACAM-5 CONCENTRATION (pg/mL)

n=3

MULTILAYER ASSEMBLED AREA ($\mu\,m^2$)

y = 37.493x + 112.93
$R^2$ = 0.9997

n=3

CEACAM-5 CONCENTRATION (pg/mL)

PROPORTION OF MULTILAYER PORTIONS

y = 0.0187x + 0.0681
$R^2$ = 0.9999

n=3

CEACAM-5 CONCENTRATION (pg/mL)

FIG.9

FIG.10

FIG.11A

FIG.11B

0.00001% ($10^{-5}$%) BSA IN DILUTION SOLUTION

PROPORTION OF MULTILAYER PORTIONS

CEACAM-5 CONCENTRATION (pg/mL)

fitting

PROPORTION OF MULTILAYER PORTIONS

$y = 0.0162x + 0.1396$
$R^2 = 0.9142$

CEACAM-5 CONCENTRATION (pg/mL)

EP 4 517 323 A1

FIG.12

CALIBRATION CURVE $\alpha$

CALIBRATION CURVE $\beta$

## FIG.13

STORED FROZEN                    NOT STORED FROZEN

Latex (2μm)

AGGREGATES OF CEACAM-5

Latex (2μm)

Latex (2μm)

MONOMER OF CEACAM-5

ANTIBODY RECOGNITION SITE

Latex (2μm)

Particle diameter (nm)
Mean: 221(Std Dev=76.9)

# FIG.14

ENLARGED VIEW OF ELISA LOW CONCENTRATION REGION

ELISA OVERALL VIEW

CEACAM-5 CONCENTRATION (pg/mL)

ERROR BARS INDICATE STANDARD DEVIATION (n = 3)

# FIG.15

AFTER DILUTING PLASMA SAMPLE BY 1000 TIMES, OPTICAL CONDENSATION DETECTION IN MICROCHANNEL ACCORDING TO PRESENT INVENTION

PLASMA CEA CONCENTRATION MEASURED BY CONVENTIONAL METHOD (MEASURED AT AICHI CANCER CENTER)

PROPORTION OF MULTILAYER PORTIONS

2.4, 4.7, 6.3, 13.2 pg/mL

n=3

UNKNOWN CONCENTRATION

ng/mL

MEASURED BY DILUTING PLASMA BY 1000 TIMES FOR OPTICAL CONDENSATION, AND MEASURED BY DILUTING PLASMA BY 10 TIMES FOR ELISA

100 TIMES MORE SENSITIVE, AND CORRELATION BETWEEN LARGE AND SMALL CONCENTRATIONS ALSO CONFIRMED

FIG.16

PHOSPHATE BUFFER: pH 7.2

PROPORTION OF MULTILAYER PORTIONS

2.4, 4.7, 6.3pg/mL

NO CEA  PLASMA A  PLASMA B  PLASMA C

PROPORTION OF MULTILAYER PORTIONS

SIGNALS INCREASED BY 3 TIMES OR MORE DUE TO BSA OPTIMIZATION

PHOSPHATE BUFFER: pH6.3

NO CEA  PLASMA A  PLASMA B

BEAD AMOUNT HALF AMOUNT

BSA 0.0001% ADJUSTED ON THAT DAY

PROPORTION OF MULTILAYER PORTIONS

NO CEA  PLASMA A  PLASMA B

EP 4 517 323 A1

FIG.17

SLIPPING PLANE

DIFFUSE LAYER

STERN LAYER

PARTICLE

potential (mV)

SURFACE POTENTIAL
ELECTRIC POTENTIAL OF
STERN LAYER
ZETA POTENTIAL

0 DISTANCE FROM
PARTICLE SURFACE

# FIG.18

EP 4 517 323 A1

FIG.19

LASER POWER
265mW, 5min

ENTIRE
ASSEMBLED
AREA ($\mu$m$^2$)

n=3

CEA (pg/mL)

LASER POWER
400mW, 5min

ENTIRE
ASSEMBLED
AREA ($\mu$m$^2$)

n=3

CEA (pg/mL)

AREA OF
MULTILAYER
PORTIONS
($\mu$m$^2$)

n=3

CEA (pg/mL)

AREA OF
MULTILAYER
PORTIONS
($\mu$m$^2$)

n=3

CEA (pg/mL)

PROPORTION
OF MULTILAYER
PORTIONS

n=3

CEA (pg/mL)

PROPORTION
OF MULTILAYER
PORTIONS

n=3

CEA (pg/mL)

# FIG.20

**ENTIRE ASSEMBLED AREA**

ASSEMBLED AREA ($\mu m^2$) ±S.D.

(Bar chart, y-axis: 0, 1,000, 2,000, 3,000; x-axis: NO CEA, PLASMA A, PLASMA B, PLASMA C, PLASMA D)

**AREA OF MULTILAYER PORTIONS**

MULTILAYER ASSEMBLED AREA ($\mu m^2$) ±S.D.

(Bar chart, y-axis: 0, 1,000, 2,000, 3,000; x-axis: NO CEA, PLASMA A, PLASMA B, PLASMA C, PLASMA D)

**AREA OF MULTILAYER PORTIONS/ ENTIRE ASSEMBLED AREA**

PROPORTION OF MULTILAYER PORTIONS ±S.D.

(Bar chart, y-axis: 0.00, 0.20, 0.40, 0.60, 0.80, 1.00; x-axis: NO CEA, PLASMA A, PLASMA B, PLASMA C, PLASMA D)

EP 4 517 323 A1

FIG.21

ENTIRE ASSEMBLED AREA

ASSEMBLED AREA ($\mu$m$^2$) ±S.D.

NO CEA   PLASMA A   PLASMA B   PLASMA C   PLASMA D

AREA OF MULTILAYER PORTIONS

MULTILAYER ASSEMBLED AREA ($\mu$m$^2$) ±S.D.

NO CEA   PLASMA A   PLASMA B   PLASMA C   PLASMA D

AREA OF MULTILAYER PORTIONS/ ENTIRE ASSEMBLED AREA

PROPORTION OF MULTILAYER PORTIONS ±S.D.

NO CEA   PLASMA A   PLASMA B   PLASMA C   PLASMA D

EP 4 517 323 A1

FIG.22

ENTIRE ASSEMBLED AREA

AREA OF MULTILAYER PORTIONS

AREA OF MULTILAYER PORTIONS/
ENTIRE ASSEMBLED AREA

# FIG.23

<u>BSA PRECOATING METHOD</u>

PTFE TUBE

"Precoating buffer"
BSA CONCENTRATION  0.0001%
$(1.0 \times 10^{-4} \% \text{ (w/v)})$
PHOSPHATE BUFFER pH6.7

CHANNEL

"MEASUREMENT SAMPLE"
ANTIBODY-MODIFIED BEADS + CEA

EP 4 517 323 A1

FIG.24

**ENTIRE ASSEMBLED AREA**

ASSEMBLED
AREA ($\mu$m$^2$)
±S.D.

CEA FINAL CONCENTRATION (pg/mL)

**AREA OF MULTILAYER PORTIONS**

MULTILAYER
ASSEMBLED
AREA ($\mu$m$^2$)
±S.D.

CEA FINAL CONCENTRATION (pg/mL)

**AREA OF MULTILAYER PORTIONS/
ENTIRE ASSEMBLED AREA**

PROPORTION OF
MULTILAYER
PORTIONS
±S.D.

$y = 0.0181x + 0.1795$
$R^2 = 0.8786$

CEA FINAL CONCENTRATION (pg/mL)

EP 4 517 323 A1

FIG.25

ENTIRE ASSEMBLED AREA

ASSEMBLED AREA ($\mu$m$^2$) ±S.D.

CEA FINAL CONCENTRATION (pg/mL)

AREA OF MULTILAYER PORTIONS

MULTILAYER ASSEMBLED AREA ($\mu$m$^2$) ±S.D.

CEA FINAL CONCENTRATION (pg/mL)

AREA OF MULTILAYER PORTIONS/ ENTIRE ASSEMBLED AREA

PROPORTION OF MULTILAYER PORTIONS ±S.D.

y = 0.0238x + 0.1161
R$^2$ = 0.975

CEA FINAL CONCENTRATION (pg/mL)

EP 4 517 323 A1

# FIG.26

WHEN VIEWED FROM ABOVE          WHEN VIEWED FROM SIDE

NUMBER RATIO IS 2 $\mu$m:1 $\mu$m = 1:8 AND
VOLUME RATIO IS ABOUT 1:1

"NUMBER RATIO IS 1:0.4" WHEN VOLUME
RATIO IS 1:0.05

LASER BEAM

BEAD
(1 $\mu$m)

BEAD
(2 $\mu$m)

MIXING RATIO OF BEADS  2 $\mu$m:1 $\mu$m = 1:0          2 $\mu$m:1 $\mu$m = 1:0.05          2 $\mu$m:1 $\mu$m = 1:0.1

EXPERIMENT B

$y = 0.0063x + 0.3022$
$R^2 = 0.1726$

PROPORTION
OF MULTILAYER
PORTIONS
±S.D.

1.00
0.80
0.60
0.40
0.20
0.00

0.0          10.0          20.0

CEA FINAL CONCENTRATION
(pg/mL)

EXPERIMENT A

$y = 0.0238x + 0.1161$
$R^2 = 0.975$

PROPORTION
OF MULTILAYER
PORTIONS
±S.D.

1.00
0.80
0.60
0.40
0.20
0.00

0.0          10.0          20.0

CEA FINAL CONCENTRATION
(pg/mL)

EXPERIMENT C

$y = 0.0109x + 0.1759$
$R^2 = 0.6085$

PROPORTION
OF MULTILAYER
PORTIONS
±S.D.

1.00
0.80
0.60
0.40
0.20
0.00

0.0          10.0          20.0

CEA FINAL CONCENTRATION
(pg/mL)

EP 4 517 323 A1

## INTERNATIONAL SEARCH REPORT

| International application No. |
| --- |
| **PCT/JP2023/018633** |

### A. CLASSIFICATION OF SUBJECT MATTER

***G01N 33/53***(2006.01)i; ***G01N 33/574***(2006.01)i; ***G01N 21/53***(2006.01)i
FI:    G01N33/53 V; G01N33/574 B; G01N21/53 Z

According to International Patent Classification (IPC) or to both national classification and IPC

### B. FIELDS SEARCHED

Minimum documentation searched (classification system followed by classification symbols)

G01N33/53; G01N33/574; G01N21/53

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| A | 藤原佳奈. マイクロ狭小空間における生物学的ナノ粒子の選択的光誘起集積化. 応用物理学会秋季学術講演会講演予稿集. 26 August 2021, vol. 82, Page.ROMBUNNO.12p-N107-10, (FUJIWARA, Kana et al. Selective Light-induced Assembly of Biological Nanoparticles in a Micro Narrow Space. Proceedings of the 82nd JSAP Autumn Meeting.) fig. 1 | 1-15 |
| A | WO 2021/040021 A1 (UNIVERSITY PUBLIC CORPORATION OSAKA) 04 March 2021 (2021-03-04) claims 1-14, paragraphs [0020]-[0029], [0039], [0070]-[0081], fig. 9-11 | 1-15 |
| A | WO 2006/129796 A1 (UNIV TOKYO AGRICULTURE & TECHNOLOGY) 07 December 2006 (2006-12-07) paragraph [0077] | 1-15 |
| A | JP 2006-242597 A (FUJI PHOTO FILM CO., LTD.) 14 September 2006 (2006-09-14) paragraph [0040] | 1-15 |
| A | JP 7-20129 A (TOKUYAMA CORP.) 24 January 1995 (1995-01-24) paragraph [0053] | 1-15 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **11 July 2023** | **01 August 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2023/018633** |

**C.  DOCUMENTS CONSIDERED TO BE RELEVANT**

| Category* | Citation of document, with indication, where appropriate, of the relevant passages | Relevant to claim No. |
| --- | --- | --- |
| A | JP 2020-509349 A (ABAXIS, INC.) 26 March 2020 (2020-03-26)<br>  claim 16 | 1-15 |
| A | WO 2012/077756 A1 (OSAKA PREFECTURE UNIVERSITY PUBLIC CORPORATION) 14 June 2012 (2012-06-14)<br>  abstract, claims, fig. 31, 32 | 1-15 |
| A | JP 2018-194550 A (OSAKA PREFECTURE UNIVERSITY PUBLIC CORP.) 06 December 2018 (2018-12-06)<br>  claim 7, paragraphs [0035], [0079], [0142], [0154], fig. 26B | 1-15 |
| A | WO 2014/192937 A1 (OSAKA PREFECTURE UNIVERSITY PUBLIC CORP.) 04 December 2014 (2014-12-04)<br>  fig. 6 | 1-15 |
| A | US 2017/0297036 A1 (CHANG GUNG UNIVERSITY) 19 October 2017 (2017-10-19)<br>  abstract | 1-15 |
| A | 光濃縮によるがんの超早期診断法の開発 ～極微量の生体マーカーを検出する革新的技術の確立へ～. 大阪府立大学LAC-SYS研究所. [online], 20 April 2021 [retrieved on 11 July 2023], Internet: <URL: https://www.osakafu-u.ac.jp/omu-content/uploads/sites/1162/pr20210420_1.pdf>, (Osaka Prefecture University, Research Institute for Light-induced Acceleration System), non-official translation (Development of Optical Condensation-Based Methods for Super-Early Cancer Diagnosis: Towards the Creation of Innovative Technologies for Detecting Trace Biomarkers.)<br>  entire text | 1-15 |
| P, A | 飯田琢也. 低侵襲ハイスループット光濃縮システムの開発. [online] 2022 [retrieved on 11 July 2023], Internet: <URL: https://projectdb.jst.go.jp/file/JST-PROJECT-21460217/JST_1150122_21460217_2021_%E9%A3%AF%E7%94%B0_PYR.pdf>, non-official translation (IIDA, Takuya. Development of Minimally Invasive High-Throughput Optical Condensation Systems.)<br>  2. Overview of R&D Results | 1-15 |
| A | 飯田琢也. 光による分子認識制御と高感度バイオセンサ応用. 中谷医工計測技術振興財団年報. 20 December 2016, no. 30, pp. 175-180, non-official translation (IIDA, Takuya. Applications of Light-Based Molecular Recognition Control and High-Sensitivity Biosensors. Annual Report of the Nakatani Foundation.)<br>  entire text, all drawings | 1-15 |
| A | 飯田琢也. 光誘導加速システム(LAC-SYS)と局在表面プラズモンの協力効果によるバイオ分析技術の開拓. プラズモニクスシンポジウム. 2019, vol. 16, p. 12, non-official translation (IIDA, Takuya. Development of Bio-Analysis Technologies Based on Results of Cooperation between Light-Induced Acceleration Systems (LAC-SYS) and Localized Surface Plasmons.)<br>  entire text | 1-15 |
| P, A | 叶田雅俊. 自己組織型ナノボウル基板によるウイルスの2ステップ光濃縮法の開発. 応用物理学会秋季学術講演会講演予稿集. 26 August 2022, vol. 83, Page.ROMBUNNO.20a-P05-3, (KANODA, Masatoshi et al. Development of Two-step Optical Condensation Method by Self-assembled Nano-bowl Substrate. Proceedings of the JSAP Autumn Meeting.)<br>  entire text, all drawings | 1-15 |

Form PCT/ISA/210 (second sheet) (January 2015)

**INTERNATIONAL SEARCH REPORT**
Information on patent family members

International application No.

**PCT/JP2023/018633**

| Patent document cited in search report | | | Publication date (day/month/year) | Patent family member(s) | | | Publication date (day/month/year) |
|---|---|---|---|---|---|---|---|
| WO | 2021/040021 | A1 | 04 March 2021 | US | 2022/0326249 | A1 | |
| | | | | claims 1, 15-28, paragraphs [0078], [0079], [0090], [0124]-[0136], fig. 9-11 | | | |
| | | | | EP | 4024030 | A1 | |
| WO | 2006/129796 | A1 | 07 December 2006 | US | 2009/0325258 | A1 | |
| | | | | paragraph [0100] | | | |
| JP | 2006-242597 | A | 14 September 2006 | US | 2006/0194887 | A1 | |
| | | | | paragraph [0049] | | | |
| JP | 7-20129 | A | 24 January 1995 | (Family: none) | | | |
| JP | 2020-509349 | A | 26 March 2020 | WO | 2018/140953 | A1 | |
| | | | | claim 16 | | | |
| | | | | US | 2022/0091113 | A1 | |
| | | | | EP | 3574308 | A1 | |
| | | | | KR | 10-2019-0112783 | A | |
| | | | | CN | 110892250 | A | |
| | | | | KR | 10-2023-0011477 | A | |
| WO | 2012/077756 | A1 | 14 June 2012 | US | 2013/0252275 | A1 | |
| | | | | abstract, fig. 31, 32, claims | | | |
| JP | 2018-194550 | A | 06 December 2018 | (Family: none) | | | |
| WO | 2014/192937 | A1 | 04 December 2014 | EP | 2993460 | A1 | |
| | | | | fig. 6 | | | |
| | | | | US | 2016/0123968 | A1 | |
| US | 2017/0297036 | A1 | 19 October 2017 | US | 2020/0009581 | A1 | |
| | | | | US | 2020/0016606 | A1 | |
| | | | | US | 2020/0016607 | A1 | |
| | | | | US | 2020/0030818 | A1 | |
| | | | | TW | 201738381 | A | |
| | | | | CN | 107304414 | A | |

Form PCT/ISA/210 (patent family annex) (January 2015)

**REFERENCES CITED IN THE DESCRIPTION**

**Patent documents cited in the description**

- WO 2014192937 A

- WO 2021040021 A